# FASCICULE DE BREVET EUROPEEN

(11) **EP 3 410 884 B1**
(45) Date de publication et mention de la délivrance du brevet: **07.06.2023**
(21) Numéro de dépôt: 17707374.9
(22) Date de dépôt: 02.02.2017
(51) Int. Cl.: G08B 6/00, G08B 21/02, G08B 25/10, H04B 1/3827

(54) **ARTICLE VESTIMENTAIRE INTELLIGENT ET COMMUNICANT, PROCÉDÉ ET INSTALLATION DE COMMUNICATION BIDIRECTIONNELLE AVEC UN TEL ARTICLE VESTIMENTAIRE**
KOMMUNIZIERENDER ARTIKEL EINES INTELLIGENTEN KLEIDUNGSSTÜCKES SOWIE VERFAHREN UND VORRICHTUNG FÜR ZWEIWEGE-KOMMUNIKATION MIT SOLCH EINEM KLEIDUNGSSTÜCK
COMMUNICANT ARTICLE OF SMART CLOTHING AND METHOD AND APPARATUS FOR TWO-WAY COMMUNICATION WITH SUCH AN ARTICLE OF CLOTHING

(30) Priorité: 05.02.2016 FR 1650956
(43) Date de publication de la demande: 12.12.2018
(73) Titulaire: Intellinium, 13587 Aix En Provence Cedex 03 (FR)
(72) Inventeur: DESTRIAN, Mathieu, 13630 Carry-Le-Rouet (FR); FRANCHETTO, Giuliano, 13850 Greasque (FR)
(74) Mandataire: Germain Maureau
(86) Numéro de dépôt international: PCT/FR2017/050229
(87) Numéro de publication internationale: WO 2017/134388

(56) Documents cités:
- WO-A2-2014/121011
- KR-A- 20140 093 807
- US-A1- 2014 135 591
- US-B1- 7 969 315

## Description

La présente invention se rapporte à un article vestimentaire permettant à un porteur d'établir une communication bidirectionnelle, c'est-à-dire dans un sens descendant et un sens montant, avec des personnes, appareils ou services distants.

L'invention concerne également un procédé de communication bidirectionnelle avec un tel article vestimentaire, au sein d'une installation comprenant un serveur central distant et au moins une passerelle (ou « gateway ») de liaison entre l'article vestimentaire et le serveur central.

Dans une application particulière et non limitative, l'invention est prévue pour permettre à un porteur d'un article vestimentaire de transmettre une alarme avertissant d'une situation de danger ou de crise et, en réponse à son alarme, de recevoir des instructions ou informations pour l'aider dans une telle situation, et/ou pour permettre à un porteur d'un article vestimentaire de recevoir une alerte signalant une situation de danger ou de crise avec éventuellement des instructions pour gérer une telle situation.

L'invention trouve une application privilégiée et non limitative dans le domaine des équipements de protection individuelle qui sont, par définition, des dispositifs ou moyens destinés à être portés ou tenus par un travailleur en vue de le protéger contre un ou plusieurs risques susceptibles de menacer sa santé ou sa sécurité, et notamment dans le domaine des chaussures de sécurité voire de manière plus large dans le domaine des chaussures.

Une telle invention peut également être appliquée pour permettre l'interaction ou la communication pour des personnes en situation de handicap, pour des joueurs de jeux vidéo immersifs, pour des membres de forces armées ou forces de police ayant besoin d'établir des transmissions discrètes sans faire appel aux mains utilisées par ailleurs, pour des membres de services publiques d'intervention comme les services de pompiers, services de secours, ou services de soins, ou pour des adultes pratiquant des jeux intimes sollicitant les sens.

Dans le secteur des dispositifs personnels ou portatifs d'alarme, il est connu d'employer des modules portatifs ou terminaux téléphoniques munis d'un bouton d'alarme, qui présentent les inconvénients d'être peu discrets et encombrants, de pouvoir être facilement oubliés, de nécessiter éventuellement l'utilisation d'un terminal téléphonique ou équivalent pour se connecter à un réseau, d'être détectables en cas de fouille et inopérants dans certaines situations (mains entravées, nécessité de discrétion, ...).

Pour répondre en partie à ces problèmes, il est connu du document EP 2 774 502 de disposer, à l'intérieur d'une chaussure de sécurité, un ou plusieurs capteurs propres à mesurer des paramètres indicatifs d'un accident de travail subi par le porteur de la chaussure (comme par exemple la détection d'une chute), un microcontrôlleur (autrement appelé en abrégé MCU) qui détermine si les paramètres mesurés traduisent un tel accident, et un émetteurrécepteur sans fil apte à transmettre automatiquement un signal d'alarme à un lecteur distant lorsque le microcontrôlleur établit une situation d'accident.

Cependant, la chaussure du document EP 2 774 502 ne permet pas à son porteur de signaler une situation de danger qui le concerne ou dont il est témoin, mais permet uniquement de signaler automatiquement un accident qu'il vient de subir. Autrement dit, le porteur ne peut pas communiquer de sa propre initiative, car seule la détermination automatique d'une situation d'accident par le microcontrôlleur sur la base des mesures des capteurs est à l'initiative de la communication avec le lecteur distant. De plus, la chaussure ne peut pas communiquer avec le porteur pour lui signaler que son signal d'alarme (automatique dans la cas présent) est bien pris en compte, et ne peut pas non plus permettre de recevoir une alerte concernant un danger immédiat ou à venir le concernant. En effet, bien que des avertisseurs sonores ou lumineux soient prévus sur la chaussure, ces derniers ne son activés par le microcontrôlleur qu'en cas de détermination automatique d'une situation d'accident, voire en cas de dysfonctionnements de l'électronique de la chaussure ou de décharge de la batterie. Ainsi, le porteur ne reçoit aucune confirmation que le signal d'alarme a bien été adressé avec succès, voire a bien été reçu, voire est bien pris en considération par les services compétents.

Il est également connu du document FR 2 156 280 d'équiper une chaussure avec un organe de commande, placé sur la semelle à l'intérieur de la chaussure, qui est constitué d'un contacteur flexible formant un interrupteur manipulable par un orteil du porteur. En cas de danger, le porteur déplace le contacteur avec son orteil afin d'établir un contact électrique qui déclenchera automatiquement l'émission d'un signal d'alarme par un émetteur logé dans le talon de la chaussure.

Cependant, une telle chaussure ne permet en aucun cas au porteur d'avoir la confirmation que le signal d'alarme a bien été adressé avec succès, voire a bien été reçu, voire est bien pris en considération par les services compétents. De manière générale, cette chaussure ne permet pas de recevoir des messages provenant de l'extérieur. Ensuite, l'emploi d'un contacteur flexible ne permet qu'un usage binaire, soit l'interrupteur est ouvert soit il est fermé, limitant ainsi son usage à un simple envoi de signal d'alarme. Enfin, cette chaussure n'intègre aucun moyen pour éviter des déclenchements parasites, intempestifs ou non désirés, autrement dit des faux positifs, de sorte que, par inadvertance, le porteur de la chaussure peut adresser un signal d'alarme sans le faire exprès et sans possibilité de savoir qu'il a adressé un tel signal d'alarme et non plus d'annuler son signal d'alarme.

L'état de la technique peut également être illustré par l'enseignement du document US 2014/135591 qui divulgue une chaussure équipée d'un capteur de pression dans le talon pour détecter la présence/absence du pied, un détecteur biologique pour mesurer une information biologique, un contrôleur qui active ou désactive le détecteur biologique en fonction de la détection de la présence/absence du pied, un émetteur/transmetteur qui permet de transmettre vers un serveur externe une information d'identification et l'information biologique issue du détecteur biologique, et pour recevoir du serveur externe un signal d'alarme généré en fonction de l'information biologique, et un avertisseur sonore qui est activé à la réception du signal d'alarme pour émettre un signal d'avertissement qui soit audible par des personnes autour du porteur afin que ces personnes soient alertées d'une situation urgente et puissent porter secours au porteur.

Bien que la chaussure du document US 2014/135591 soit intéressante en cas de problème de santé du porteur, cette chaussure ne permet en aucune façon au porteur de communiquer de sa propre initiative un message vers l'extérieur, et elle ne permet pas non plus au porteur de recevoir des messages intelligibles. De plus, la chaussure ne peut pas permettre de recevoir une alerte autre que celle relative à son état de santé personnel. En effet, bien qu'un avertisseur sonore soit prévu sur la chaussure, ce dernier n'est activé par le contrôlleur qu'en cas de détermination par le serveur d'un souci de santé, et rien de plus.

Il est aussi connu du document KR 2014 0093807 A d'intégrer dans un gant un module de radiocommunication, un capteur de pression à base d'un caoutchouc conducteur, une unité de contrôle, une batterie et un module haptique, permettant notamment d'envoyer un code morse vers l'extérieur. Cependant, une telle solution ne permet pas une communication bidirectionnelle de messages intelligibles entre le porteur du gant et l'extérieur.

La présente invention a pour but de résoudre en tout ou partie les inconvénients précités, en proposant un article vestimentaire qui permet une communication bidirectionnelle entre le porteur et au moins un tiers externe (comme par exemple une personne porteuse d'un article vestimentaire similaire, un service externe via un serveur central, un appareil connecté communiquant, ...), à l'initiative du tiers externe ou à l'initiative du porteur, pour échanger des messages en sens montant et descendant, étant entendu qu'une telle communication bidirectionnelle permettra par exemple au tiers externe de signaler au porteur que son message a bien été reçu et qu'il est en cours de traitement, ou bien au porteur de signaler au tiers externe qu'il a bien pris en compte son message.

L'invention permet également au porteur de transmettre des messages plus complexes qu'un simple signal d'alarme, par exemple sous la forme de séquences codées prédéfinies et/ou de messages en morse.

L'invention a en outre pour objectif de permettre au porteur de l'article vestimentaire de recevoir des messages provenant d'autres porteurs d'articles vestimentaires similaires et/ou d'un système informatisé embarqué dans un objet ou appareil et/ou d'une application disponible sur un équipement portable (téléphone, montre, ...) et/ou d'un programme disponible sur un serveur informatique local ou distant.

A cet effet, elle propose un article vestimentaire selon la revendication 1.

Le capteur de force, qui est un dispositif utilisé pour convertir une séquence d'un ou plusieurs efforts successifs (en l'occurrence une séquence de plusieurs pressions successives) appliquée par le porteur (avec une main, un pied, un doigt, un orteil, ou une autre partie du corps selon le type d'article vestimentaire) sur le capteur en une variation de signal électrique, et notamment en une variation de résistivité électrique, permet ainsi au porteur d'émettre un signal dit porteur plus ou moins complexe. Il suffit au porteur d'appliquer sur le capteur de force une séquence prédéfinie d'efforts successifs (un effort pouvant être plus ou moins long et/ou plus ou moins rapproché temporellement de l'effort précédent et de l'effort suivant et/ou exercé avec une plus ou moins grande pression) qui sera traduit par le contrôleur en un signal porteur associé à un message prédéfini qui aura du sens pour les services externes qui réceptionneront ce signal porteur. Il est également possible d'établir une communication tout aussi complexe entre le contrôleur de la chaussure et le porteur (notamment pour demander des confirmations avant d'alerter les services externes).

Pour limiter, voire éviter, les faux-positifs, le ou les capteurs de force sont avantageusement associés à :
- une instrumentation électronique à base d'amplificateurs opérationnels ; et/ou
- un module de prétraitement et de filtrage pour prétraiter et filtrer les données de mesure issues du ou des capteurs, avant toute analyse par le centre de traitement numérique permettant d'interpréter la séquence d'efforts successifs jouées par le porteur.

Chaque séquence prédéfinie d'efforts successifs peut correspondre à une sorte de raccourci pour un message donné (par exemple « alerte », « attention hommes armés », « incident d'une nature spécifique », « je ne peux pas parler », « je suis kidnappé », « je suis blessé », « je suis agressé », « je vous informe d'un danger », « je soupçonne une personne d'être un terroriste », etc.) ou bien peut correspondre à une séquence en alphabet morse et/ou dans un autre alphabet ou code de langage qui pourrait être configuré dans le centre de traitement numérique. Il est à noter que tels raccourcis peuvent être paramétrés par le porteur et/ou par une personne tierce (par exemple un membre de l'organisation encadrant le porteur). De plus, comme indiqué ci-dessus, le porteur peut effectuer des séquences prédéfinies d'efforts successifs en alphabet morse, en agissant sur le(s) capteur(s) de force, afin également d'émettre des messages complexes.

De plus, les services externes pourront adresser un message au porteur de la chaussure, sous la forme d'un signal dit d'avertissement, qui sera communiqué au porteur via le(s) avertisseur(s) ; un tel message ayant un sens bien précis (selon le choix du ou de l'avertisseur et selon la forme du signal d'avertissement) qui sera ensuite interprété par le porteur, avec par exemple comme signification « message bien reçu », « envoi des services en cours », « rendez-vous à tel endroit », « appelez immédiatement votre manager », « quittez immédiatement cette zone », ... Il est également envisageable qu'un tiers externe (comme par exemple une personne porteuse d'un article vestimentaire similaire, un service externe, un appareil connecté communiquant, ...) adresse un message complexe qui se traduira par un signal d'avertissement en alphabet morse qui sera compris par le porteur ayant des connaissances en alphabet morse.

Les signaux d'avertissement, qui ont chacun un sens précis, peuvent se distinguer en jouant sur l'amplitude et/ou l'intensité et/ou la fréquence et/ou les largeurs d'impulsion avec une modulation de largeur d'impulsions (autrement appelé « Pulse Width Modulation » ou PWM).

Il est entendu au sens de la présente invention, par article vestimentaire, un article prévu pour être porté comme vêtement par un porteur, comme par exemple, à titre non limitatif, un vêtement de buste (tee-shirt, pull, veste, maillot de corps, sous-pull, débardeur, ...), un sous-vêtement (slip, caleçon, boxer, culotte, soutien-gorge, ...), un vêtement de mains (gant, mitaine, moufle), un vêtement de jambes (pantalon, short, jupe, ...), un vêtement de pieds (chaussure, sandale, ...) ou un vêtement de tête (chapeau, bonnet, cagoule, ...).

Il est également bien compris qu'un module de radiocommunication bidirectionnelle intègre un émetteur/récepteur (ou « transceiver » en anglais) pour une communication en sens montant et en sens descendant.

De manière avantageuse, le centre de traitement numérique intègre au moins un microprocesseur associé à au moins un microcontrôleur.

Sans que cela soit limitatif, le centre de traitement numérique intègre au moins un composant FPGA (« Field-Programmable Gate Array ») et/ou au moins un composant DSP (« Digital Signal Processing »).

Une telle solution permet d'exploiter au moins un microprocesseur pour les tâches complexes et énergivores, et de réserver au moins à un microcontrôleur les tâches peu consommatrices en énergie, celles nécessitant l'acquisition de signaux numériques ou analogiques extérieurs et celles pouvant réveiller le microprocesseur lorsque nécessaire.

L'intérêt du duo microprocesseur (MPU) / microcontrôleur (MCU) réside aussi dans la capacité de faire s'exécuter plusieurs programmes complexes sur le microprocesseur ayant-lui-même une capacité de pouvoir héberger un système d'exploitation évolué.

D'autre part, ce duo permet une gestion découplée des modules de radiocommunication en fonction de la consommation de ces derniers ainsi que de leur protocole de radiocommunication.

Par exemple, le microcontrôleur va piloter le(s) module(s) de radiocommunication dits « connection-less » (par exemple module de radiocommunication sous protocole LoRa^{™}) et le microprocesseur va piloter le(s) module(s) de radiocommunication dits « IP » (par exemple module de radiocommunication sous protocole GSM^{™} ou Wi-Fi^{™} ou Li-Fi^{™}).

Selon une caractéristique, l'article vestimentaire comprend au moins un avertisseur du type vibreur placé à l'intérieur de l'article vestimentaire afin d'émettre un signal d'avertissement vibratoire apte à être ressenti par le porteur.

L'emploi d'un ou plusieurs vibreurs est particulièrement avantageux pour communiquer, en toute discrétion, des messages au porteur, plus ou moins complexes, sous la forme de signaux d'avertissement vibratoires ; chaque signal vibratoire pouvant se présenter sous la forme d'une séquence prédéfinie de vibrations successives (une vibration pouvant être plus ou moins longue et/ou plus ou moins rapproché temporellement de la vibration précédente et de la vibration suivante) qui sera traduit par le porteur comme un message prédéfini ayant une signification précise.

Chaque séquence prédéfinie de vibrations successives peut correspondre à une sorte de raccourci pour un message donné ou bien peut correspondre à une séquence en alphabet morse et/ou dans un autre alphabet ou code de langage qui pourrait être configuré dans le centre de traitement numérique. Il est à noter que tels raccourcis peuvent être paramétrés par le porteur et/ou par une personne tierce.

Il est à noter que de tels signaux d'avertissement vibratoires peuvent être utilisés comme des retours vibratoires (ou retours haptiques) dans le cadre de l'interaction entre un programme embarqué dans le centre de traitement numérique de l'article vestimentaire et le porteur, comme par exemple la confirmation de l'exécution d'une commande.

Avantageusement, l'article vestimentaire comprend plusieurs vibreurs placés à différentes positions spatiales à l'intérieur de l'article vestimentaire, afin d'émettre des signaux d'avertissement vibratoires respectifs aptes à être ressentis par différentes zones du corps du porteur pour être interprétés spatialement par le porteur.

Grâce à une telle distribution spatiale des vibreurs à l'intérieur de l'article vestimentaire, il est possible d'adresser des messages plus complexes au porteur, et notamment des messages de guidage dans l'espace, afin de conduire le porteur vers une destination donnée.

Selon une autre caractéristique, l'article vestimentaire comprend :
- au moins un avertisseur du type source lumineuse placée sur l'extérieur de l'article vestimentaire afin d'émettre un signal d'avertissement lumineux apte à être vu (et interprété) au moins par le porteur (et éventuellement par toute personne proche du porteur) ; et/ou
- au moins un avertisseur du type source sonore afin d'émettre un signal d'avertissement sonore apte à être entendu (et interprété) au moins par le porteur (et éventuellement par toute personne proche du porteur) ; et/ou
- au moins un avertisseur du type source odorante afin d'émettre un signal d'avertissement odorant apte à être senti olfactivement (et interprété) au moins par le porteur (et éventuellement par toute personne proche du porteur).

De tels avertisseurs, moins discrets que le(s) vibreur(s), permettent d'adresser d'autres messages au porteur et/ou au(x) éventuelles personne(s) proche(s), enrichissant ainsi la communication entre le porteur et les tiers externes ; étant noté que de tels avertisseurs peuvent être désactivés dans certaines situations signalées par le porteur, comme par exemple une situation de kidnapping, de présence d'assaillants, de présence sur zone de combat, ...

Dans une réalisation particulière, le centre de traitement numérique intègre une première table de conversion, notamment du type paramétrable, configurée pour convertir des séquences prédéfinies d'efforts successifs appliqués par le porteur sur le capteur de force en des signaux porteurs respectifs associés à des messages.

Ainsi, une telle première table de conversion permet de convertir les séquences d'efforts successifs du porteur en des messages lisibles par les tiers externes ; étant noté que le porteur peut au préalable paramétrer cette première table de conversion pour définir des raccourcis (autrement dit des séquences spécifiques) pour adresser des messages de son choix, ou pour décoder des séquences en alphabet morse (dans ce cas la première table de conversion intègre une table d'alphabet morse).

Il est à noter qu'à chaque séquence d'efforts successifs reconnue par le centre de traitement numérique dans la première table de conversion (que ce soit une séquence de raccourci ou une séquence en alphabet morse) correspond un signal porteur propre. La différence entre les différents signaux porteurs peut se faire de différentes manières, comme par exemple et à titre non limitatif, en jouant sur la durée, l'amplitude, la fréquence, la modulation, ...

Avantageusement, le centre de traitement numérique intègre une seconde table de conversion, notamment du type paramétrable, configurée pour convertir des signaux externes prédéfinis en des motifs respectifs d'émission d'au moins un signal d'avertissement.

Ainsi, une telle seconde table de conversion permet de convertir les messages adressés par les services externes en des signaux d'avertissement donnés ; étant noté que le porteur peut au préalable paramétrer cette seconde table de conversion pour définir des raccourcis (autrement dit des motifs spécifiques de signaux d'avertissement) pour comprendre les messages qui lui sont adressés.

Dans un mode de réalisation particulier, l'article vestimentaire est une chaussure comprenant une semelle et une partie supérieure de chaussage pourvue d'une entrée pour l'introduction d'un pied d'un porteur.

De manière avantageuse, le ou chaque capteur de force est positionné sur une face interne de la partie supérieure de chaussage.

Le placement du au moins un capteur de force sur la partie supérieure de chaussage, et non sur ou dans la semelle, permet d'éviter des faux positifs (autrement dit des messages effectués par erreur), le pied étant en constant appui sur la semelle, garantissant l'envoi de signaux porteurs voulus par le porteur. Un tel placement permet en outre de limiter la consommation électrique et de limiter l'usure du capteur de force dans le temps.

Cependant, dans le cadre de la présente invention, il aurait été envisageable d'intégrer le ou les capteurs de force sur ou dans la semelle.

De manière préférentielle, le ou chaque capteur de force est positionné sur une face interne de la partie supérieure de chaussage et à l'avant de la chaussure, de sorte que le ou chaque capteur de force est placé au-dessus d'au moins un orteil du pied.

Une telle localisation est particulièrement avantageuse, car à cet endroit (au-dessus des orteils du pied), le pied n'est pas en contact avec la partie supérieure de chaussage, et donc ne sera pas en contact avec le(s) capteur(s) de force, de sorte que seule une poussée verticale des orteils (ou du seul gros orteil selon le positionnement latéral) pourra générer un signal porteur, évitant ainsi les faux signaux non désirés.

Selon une possibilité de la chaussure, la batterie (et son éventuelle antenne de rechargement) sont situées au niveau du talon de la chaussure.

Selon une autre possibilité de la chaussure, la chaussure comprend en outre une coque de sécurité placée à l'avant de la chaussure, et le ou chaque capteur de force est disposé sous ladite coque de sécurité.

Ainsi, la chaussure, du type chaussure de sécurité, présente une coque qui protégera les doigts de pied mais également le capteur de force qui est à la base de l'émission de signaux porteurs par le porteur.

Avantageusement, la chaussure comprend plusieurs vibreurs placés respectivement à l'avant, à l'arrière, sur le dessus, sur le dessous et sur les côtés droite et gauche de la chaussure afin d'émettre des signaux d'avertissement vibratoires respectifs aptes à être ressentis par différentes zones du pied.

Selon une autre possibilité de la chaussure, la semelle comprend une couche de semelle extérieure et une couche de semelle intérieure encadrant une couche d'isolation en mousse, notamment en mousse polyuréthane, où le centre de traitement numérique et le module de radiocommunication sont noyés à l'intérieur de ladite couche d'isolation.

Ainsi, la couche d'isolation va garantir protection et étanchéité pour le centre de traitement numérique et le module de radiocommunication, et éventuellement pour la batterie.

De plus, la chaussure pourra également comprendre, connecté au centre de traitement numérique, un détecteur de présence du pied à l'intérieur de la chaussure, permettant ainsi de réveiller le centre de traitement numérique uniquement lorsqu'un pied est à l'intérieur de la chaussure.

Il est bien noté qu'un tel détecteur de présence du pied est distinct du capteur de force employé pour permettre au porteur d'effectuer des séquences d'efforts successifs sur le capteur de force afin de permettre au porteur de communiquer des messages.

En variante ou en complément, le centre de traitement numérique, le module de radiocommunication et éventuellement la batterie peuvent être placés à l'intérieur d'un sarcophage, permettant de pouvoir récupérer et recycler les composants électroniques pour des raisons économiques et réglementaires.

Dans une réalisation particulière, la ou les antennes associées au(x) modules de radiocommunication comprennent en partie les éléments métalliques, et notamment ferreux, de la chaussure (comme par exemple une coque de sécurité, une lame métallique d'anti-perforation insérée dans la semelle, ...).

Dans une première réalisation, dite d'écoute synchrone, le centre de traitement numérique est dans une phase d'écoute continue, durant laquelle le centre de traitement numérique ouvre au moins un canal de communication entrant pour la réception de tout message entrant qui lui est adressé par l'extérieur.

Dans une seconde réalisation, dite d'écoute asynchrone, le centre de traitement numérique alterne successivement entre :
- des phases d'écoute durant lesquelles le centre de traitement numérique ouvre au moins un canal de communication entrant pour la réception de tout message entrant qui lui est adressé par l'extérieur ;
- des phases de pause durant lesquels le centre de traitement numérique ferme son ou tous ses canaux de communication entrant.

Ainsi, que ce soit dans une écoute synchrone ou asynchrone, le centre de traitement numérique de l'article vestimentaire peut réceptionner régulièrement (en continue ou avec un décalage) tout message entrant, sans qu'il soit nécessaire que le porteur de l'article vestimentaire ait émis précédemment un message vers l'extérieur. Autrement dit, le centre de traitement numérique de l'article vestimentaire peut réceptionner régulièrement tout message entrant adressé à l'initiative d'un émissaire externe (machine ou personne extérieure), par exemple pour l'informer d'une situation spécifique qui nécessite son attention.

Conformément à une autre caractéristique avantageuse de l'invention, l'article vestimentaire comprend un module de radiocommunication du type puce de radiocommunication longue portée selon une technologie LPWAN «Low Power Wide Area Network» ou LTN «Low Throughput Network».

L'emploi d'une telle technologie LPWAN ou LTN, généralement utilisée dans le secteur des objets connectés (ou loT pour Internet of Things), présente l'avantage de procurer une communication basse consommation, avec une portée maximale comprise entre plusieurs centaines de mètres jusqu'à quelques kilomètres.

Dans une réalisation particulière, l'article vestimentaire comprend un module de radiocommunication du type puce de radiocommunication courte portée (par exemple une portée maximale comprise entre un et cinq mètres, voire une quinzaine de mètres) selon une technologie BAN (Body Area Network) ou BSN (Body Sensor Network), afin d'établir une radiocommunication entre la chaussure et au moins un dispositif portatif porté par le porteur.

Selon une possibilité, le dispositif portatif est équipé d'au moins un capteur et/ou d'au moins un actionneur et/ou d'au moins un avertisseur. Ainsi, l'article vestimentaire pourra communiquer avec un ou plusieurs dispositifs portatifs au sein d'un réseau BAN, de tels dispositifs portatifs comprenant par exemple au moins un capteur suivant un paramètre vital du corps ou l'environnement du porteur (environnement chimique, thermique, physique, hydrométrique, imagerie ou vidéo ...) et/ou au moins un actionneur ou avertisseur (haut-parleur, écran, enregistreur audio et/ou vidéo...). Ainsi, l'article vestimentaire pourra recevoir les données de mesure du ou des capteurs de ce(s) dispositif(s) portatif(s) et servir de passerelle pour rediriger ces données de mesure vers les services externes.

Selon une autre possibilité, le dispositif portatif est équipé d'une étiquette de radio-identification courte-portée, comme par exemple une étiquette passive RFID (ou « passive RFID tag »), une étiquette active RFID (ou « active RFID tag ») ou une puce NFC (ou « NFC tag »), où cette étiquette de radio-identification stocke un identifiant et éventuellement d'autres données. Ainsi, l'article vestimentaire pourra identifier le dispositif portatif par une lecture de contrôle courte-portée. De cette manière, il est possible d'équiper tout ou partie des dispositifs portatifs prévus pour être portés par le porteur de l'article vestimentaire, tels que des vêtements, des outils, des éléments de protection (casque, lunettes, gants, ...), des armes, etc. Alors, l'article vestimentaire réceptionnera par lecture courte-portée les identifiants des étiquettes de radio-identification des différents dispositifs portatifs portés par le porteur, et il sera ainsi possible de vérifier si le porteur n'a pas omis (ou oublié) de s'équiper de tel ou tel dispositif portatif, ou si le porteur porte un dispositif portatif non conforme, ou si le dispositif portatif est adapté à la zone dans laquelle il se trouve à un moment donné, ou si le dispositif portatif est adapté à au moins une consigne prédéfinie, telles qu'une consigne de sécurité, une consigne d'hygiène, une consigne de maintenance industrielle ou une consigne d'installation. Cette vérification peut être effectuée soit directement par le centre de traitement numérique de l'article vestimentaire, soit par un serveur distant suite à l'envoi par le centre de traitement numérique des identifiants lus.

Dans le cas d'une omission d'un dispositif portatif (parce que son identifiant n'a pas été relevé lors de la lecture de contrôle), il pourra être envisagé d'adresser un signal d'avertissement, via au moins un avertisseur, pour informer le porteur de cette omission.

Dans le cas d'une non conformité d'un dispositif portatif (parce que son identifiant a été relevé lors de la lecture de contrôle et que la vérification établit que cet identifiant est associé à un dispositif portatif non conforme), il pourra être envisagé d'adresser un signal d'avertissement, via au moins un avertisseur, pour informer le porteur de cette non conformité.

Dans le cas d'un dispositif portatif non adapté à la zone dans laquelle il se trouve à un moment donné ou non adapté à au moins une consigne prédéfinie, il pourra être envisagé d'adresser un signal d'avertissement, via au moins un avertisseur, pour informer le porteur de cette non adaptation.

Dans une réalisation particulière, l'article vestimentaire comprend un dispositif d'affichage (par exemple un écran LED flexible) piloté par le centre de traitement numérique pour pouvoir modifier les motifs externes de l'article vestimentaire en fonction d'événements internes, de messages reçus ou de toute information reçue par le centre de traitement numérique.

Il est envisageable que ce dispositif d'affichage soit externe à l'article vestimentaire, tout en étant toujours piloté par le centre de traitement numérique de l'article vestimentaire via une connexion établie grâce au module de radiocommunication.

La présente invention se rapporte également à une installation de communication bidirectionnelle avec au moins un porteur d'un article vestimentaire conforme à l'invention, ladite installation comprenant un serveur central distant et au moins une passerelle de liaison entre un module de radiocommunication de l'article vestimentaire et le serveur central.

De manière avantageuse, une passerelle est en liaison avec le module de radiocommunication de l'article vestimentaire sur un réseau LPWAN «Low Power Wide Area Network» ou LTN «Low Throughput Network», qui est pour rappel un réseau basse consommation.

La passerelle est ensuite en communication avec le serveur central via un réseau haut débit à haute ou moyenne consommation, comme exemple un réseau filaire Ethernet ou un réseau sans fil 3G, 4G, 5G ou Wi-Fi, voire Li-Fi. Cette passerelle peut bien entendu être également connectée à un ou plusieurs autres articles vestimentaires similaires locaux, autrement dit à plusieurs articles vestimentaires à portée de la passerelle.

Selon une possibilité, le module de radiocommunication de l'article vestimentaire est en liaison avec le module de radiocommunication d'au moins un autre article vestimentaire conforme à l'invention, soit directement soit par l'intermédiaire de la passerelle.

A titre d'exemple d'une liaison directe entre les modules de radiocommunication de deux articles vestimentaires, il est envisageable d'exploiter les modules de radiocommunication pour former un réseau maillé local, ou réseau Mesh, en mettant en oeuvre, comme méthode de connexion décentralisée, une topologie de maillage où les noeuds du réseau sont les modules de radiocommunication des articles vestimentaires qui sont reliés les uns avec au moins une partie des autres de manière décentralisée.

L'intérêt d'un tel réseau maillé est de permettre une communication entre plusieurs porteurs d'articles vestimentaires, notamment par rebond d'un noeud à l'autre, dans des zones isolées ou des zones dans lesquelles les réseaux de télécommunication ont du mal à pénétrer. Dans le réseau maillé, un module de radicommunication peut être en liaison directe avec tout ou partie des autres modules de radicommunication, selon les distances entre les porteurs.

Selon une autre possibilité, l'installation comprend en outre au moins un appareil connecté équipé d'au moins un capteur de mesure d'un paramètre, notamment du type paramètre physique, chimique ou environnemental, et/ou d'au moins un actionneur, ledit appareil connecté comprenant en outre un module de radiocommunication bidirectionnelle apte à émettre des données de mesure de son capteur et/ou recevoir des données de pilotage de son actionneur, et dans laquelle le module de radiocommunication de l'article vestimentaire est en liaison avec le module de radiocommunication du au moins un appareil connecté, soit directement soit par l'intermédiaire de la passerelle.

Ainsi, l'article vestimentaire peut communiquer avec cet appareil connecté, afin par exemple de :
- recevoir des signaux externes de la part de cet appareil connecté, comme par exemple un signal externe signalant un danger reconnu par l'appareil (par exemple « attention appareil en surchauffe », « attention détection d'une fuite de gaz », ...), suite à une mesure par son capteur d'un paramètre au-delà ou en-deçà d'un seuil donné ; et/ou
- transmettre un signal porteur qui formera un signal de pilotage reçu par l'appareil connecté et déclenchant son actionneur, comme par exemple une ouverture de porte, une activation d'une aspiration, une activation d'une sirène, etc.

Selon une autre possibilité, l'installation comprend en outre au moins un dispositif portatif porté par le porteur de l'article vestimentaire, ledit dispositif portatif intégrant :
- au moins un capteur de mesure d'un paramètre, notamment du type paramètre mécanique, physique, chimique, environnemental ou physiologique, et/ou
- au moins un actionneur ou avertisseur, notamment du type unité d'enregistrement audio et/ou vidéo, source sonore, source olfactive, source vibratoire, émetteur visuel tel qu'un écran ;
ledit dispositif portatif intégrant en outre au moins une puce de radiocommunication courte portée selon une technologie WBAN «Wireless Body Area Network» ou WBASN «Wireless Body Area Sensor Network», et dans laquelle ladite puce de radiocommunication courte portée du dispositif portatif est en liaison avec la puce de radiocommunication courte portée de l'article vestimentaire.

De cette manière, le centre de traitement numérique de l'article vestimentaire peut récupérer des données provenant du dispositif portatif, et peut transférer ces données vers un tiers externe (l'article vestimentaire opérant ainsi comme une passerelle de transfert ou « gateway ») et/ou peut les analyser pour émettre des signaux d'avertissement à destination du porteur.

L'invention concerne aussi un procédé de communication bidirectionnelle avec un article vestimentaire conforme à l'invention, comprenant les étapes suivantes :
- détection d'une séquence d'efforts successifs effectuée par un porteur de l'article vestimentaire sur le au moins un capteur de force ;
- réception par le centre de traitement numérique d'un signal de détection de ladite séquence d'efforts successifs par le au moins un capteur de force, après un éventuel prétraitement et un filtrage des signaux issus directement du au moins un capteur de force ;
- analyse par le centre de traitement numérique dudit signal de détection afin de vérifier s'il est associé à un signal porteur prédéfini correspondant à un message intelligible ;
- pilotage par le centre de traitement numérique d'au moins un avertisseur pour émettre un signal d'avertissement suite à la réception dudit signal de détection ;
- pilotage par le centre de traitement numérique du module de radiocommunication pour émettre ledit signal porteur ;
- réception d'un signal externe par le module de radiocommunication, et transmission dudit signal externe au centre de traitement numérique ;
- pilotage par le centre de traitement numérique d'au moins un avertisseur pour émettre un signal d'avertissement suite à la réception dudit signal externe.

Dans une première réalisation, dite d'écoute synchrone, le centre de traitement numérique est dans une phase d'écoute continue, durant laquelle le centre de traitement numérique ouvre au moins un canal de communication entrant pour la réception de tout message entrant qui lui est adressé par l'extérieur.

Dans une seconde réalisation, dite d'écoute asynchrone, dans le cadre de procédé de communication bidirectionnelle, le centre de traitement numérique alterne successivement entre :
- des phases d'écoute durant lesquelles le centre de traitement numérique ouvre au moins un canal de communication entrant pour la réception de tout message entrant qui lui est adressé par l'extérieur ;
- des phases de pause durant lesquels le centre de traitement numérique ferme son ou tous ses canaux de communication entrant.

Selon une possibilité, que ce soit dans une écoute synchrone ou asynchrone, ce procédé de communication bidirectionnelle comprend les étapes successives suivantes :
- durant la ou une phase d'écoute, le centre de traitement numérique réceptionne le message d'annonce en provenance d'un émissaire externe ;
- le centre de traitement numérique pilote au moins un avertisseur, afin de générer un motif d'annonce spécifique d'émission d'au moins un signal d'avertissement ;
- le centre de traitement numérique réceptionne un signal de détection issu du au moins un capteur de force suite à la réalisation, par le porteur de l'article vestimentaire, d'une séquence d'efforts successifs sur le au moins un capteur de force et qui correspond à une séquence haptique d'accusé réception ;
- le centre de traitement numérique convertit le signal de détection en un message d'accusé réception ;
- le centre de traitement numérique communique à émissaire externe le message d'accusé réception.

Ainsi, l'émissaire externe, qui a pris l'initiative d'adresser un message d'annonce à l'article vestimentaire, et plus spécifiquement à son porteur, va recevoir un message d'accusé réception qui lui confirmera que le porteur a bien reçu (et compris) le message d'annonce.

D'autres caractéristiques et avantages de la présente invention apparaîtront à la lecture de la description détaillée ci-après, d'un exemple de mise en oeuvre non limitatif, faite en référence aux figures annexées dans lesquelles :
- la figure 1 est une vue schématique en perspective et en transparence partielle d'un article vestimentaire de type chaussure conforme à l'invention ;
- la figure 2 est une vue schématique d'une installation de communication bidirectionnelle avec la chaussure de la figure 1 ;
- la figure 3 est un synoptique d'une première phase de communication entre une première chaussure et une première passerelle ;
- la figure 4 est un synoptique d'une deuxième phase de communication, qui suit la première phase de la figure 3, entre la première chaussure et une seconde chaussure locale via la première passerelle ;
- la figure 5 est un synoptique d'une troisième phase de communication, qui suit la première phase de la figure 3, entre la première chaussure et une seconde chaussure distante via la première passerelle, un serveur central et une seconde passerelle ;
- la figure 6 est un synoptique d'une quatrième phase de communication, qui suit la première phase de la figure 3, entre la première chaussure et un service public (type pompier, police, santé, ...) via la première passerelle et le serveur central ;
- la figure 7 est un synoptique d'une cinquième phase de communication, qui suit la première phase de la figure 3, entre la première chaussure et des personnes (type responsable hiérarchique ou acteur de la sécurité) via la première passerelle et le serveur central ;
- la figure 8 un synoptique d'une sixième phase de communication, qui suit la première phase de la figure 3, entre la première chaussure et un premier appareil connecté via la première passerelle, et entre la première chaussure et un second appareil connecté via la première passerelle et le serveur central ;
- la figure 9 est un synoptique d'une septième phase de communication entre le serveur central et la première chaussure via la première passerelle ; et
- la figure 10 est un synoptique d'une huitième phase de communication entre un appareil connecté et la première chaussure.

La description détaillée qui suit concerne un article vestimentaire de type chaussure 1 conforme à l'invention, destinée à être portée au pied par un porteur ; étant rappelé que l'invention ne se limite pas à une telle chaussure, mais peut être envisagée avec d'autres articles vestimentaires (vêtements de buste, vêtement de mains, sous-vêtement, vêtement de tête, ...), c'est-à-dire que les différents composants électroniques décrits ci-après pourraient être intégrés à un autre type d'article vestimentaire, et pas uniquement à la chaussure 1 décrite ci-après.

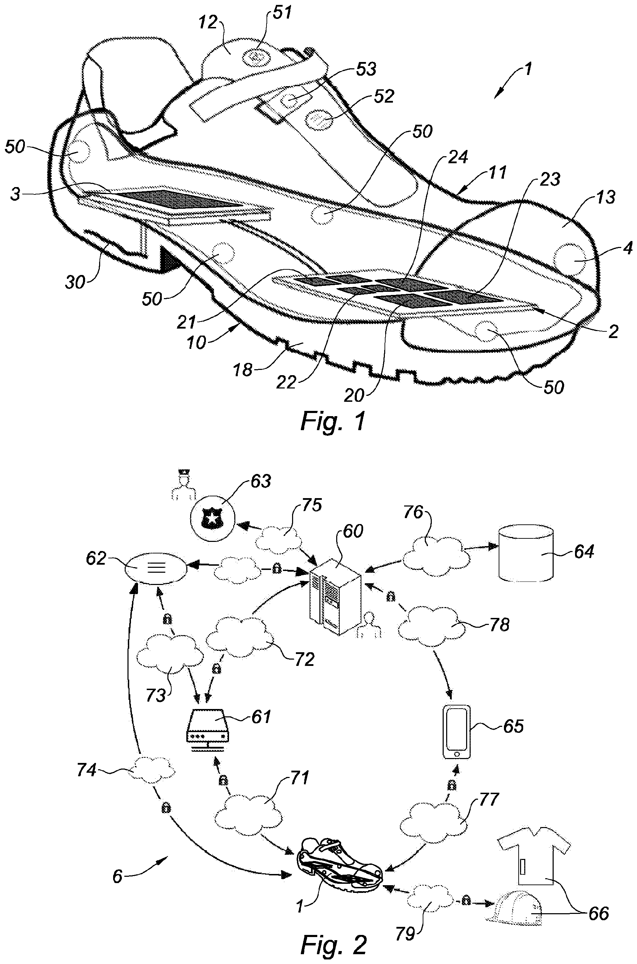

En référence à la figure 1, cette chaussure 1 conforme à l'invention comprend une semelle 10 et une partie supérieure de chaussage 11 (également appelée tige) pourvue d'une entrée pour l'introduction d'un pied d'un porteur, ainsi qu'éventuellement une languette 12 placée sur le dessus de la partie supérieure de chaussage 11 et notamment sous une zone de laçage ou plus généralement de serrage de la chaussure 1.

La semelle 10 comprend une couche de semelle extérieure 18 (qui vient en contact avec le sol) et une couche de semelle intérieure (non illustrée, qui est en contact avec le pied), ainsi qu'une couche d'isolation (non illustrée) intercalée entre la couche de semelle extérieure 18 et la couche de semelle intérieure. Cette couche d'isolation est notamment réalisée en mousse, comme par exemple en mousse polyuréthane.

La chaussure 1 illustrée sur la figure 1 est une chaussure de sécurité, catégorisée dans les équipements de protection individuelle, et intègre à ce titre une coque de sécurité 13 placée à l'avant de la chaussure 1, et plus spécifiquement à l'intérieur de la partie supérieure de chaussage 11, afin de protéger les orteils du pied. Cette coque de sécurité 13 est réalisée à titre d'exemple dans un matériau métallique, un matériau composite ou un matériau plastique.

La chaussure 1 intègre intérieurement une carte électronique 2 placée dans la semelle 10, et plus spécifiquement noyée dans sa couche d'isolation, notamment au niveau de l'avant de la chaussure 1. En variante, la carte électronique 2 pourrait être placée sensiblement au niveau du milieu de la semelle 10, voire au niveau de la voûte plantaire, en particulier au niveau du décochement entre le talon et le plat de semelle.

La carte électronique 2 intègre un centre de traitement numérique 20 reprogrammable et configurable, et qui comprend un microprocesseur (autrement appelé en abrégé MPU ou microprocesseur électronique ou processeur de signal numérique), et/ou un microcontrôleur (autrement appelé en abrégé MCU). La carte électronique 2 intègre également au moins une mémoire vive 21 (ou mémoire RAM) et au moins une mémoire Flash 22 connectées au centre de traitement numérique 20.

La carte électronique 2 intègre un module de prétraitement et de filtrage, en l'occurrence un module hardware, qui permet de pré-traiter et filtrer les données de mesure issues du ou des capteurs de force 4 décrits ultérieurement.

La carte électronique 2 intègre un module de radiocommunication 23 bidirectionnelle apte à émettre et recevoir sans fil des signaux, où ce module de radiocommunication 23 est du type puce de radiocommunication longue portée selon une technologie LPWAN «Low Power Wide Area Network» ou LTN «Low Throughput Network».

A titre d'exemple non limitatif, parmi les différents protocoles de technologie LPWAN ou LTN, peuvent être retenus les protocoles LoRa^{™}, Sigfox^{™}, NWave^{™}, Neul^{™}, OnRamp^{™}, 5G^{™}, Platanus^{™}, Telensa^{™} et Weightless^{™}.

La puce de radiocommunication longue portée 23 est connectée au centre de traitement numérique 20, et plus spécifiquement au microprocesseur qui le pilote, ainsi qu'à une antenne (non illustrée) disposée dans la chaussure 1.

La carte électronique 2 intègre également un autre module de radiocommunication 24 bidirectionnelle du type puce de radiocommunication courte portée selon une technologie WBAN «Wireless Body Area Network» ou WBASN «Wireless Body Area Sensor Network», afin de pouvoir établir une radiocommunication sur un réseau WBAN ou WBASN entre la chaussure 1 et un terminal mobile 65 et/ou au moins un dispositif portatif 66 (visible sur la figure 2) porté par le porteur et équipé d'au moins un capteur.

A titre d'exemple non limitatif, parmi les différents protocoles de technologie WBAN ou WBASN, peuvent être retenus les protocoles Zigbee^{™} et Bluetooth^{™} Low Energy (BLE), ainsi que les standards IEEE 802.15 dont les standards IEEE 802.15.4 et IEEE 802.15.6, ainsi qu'un protocole RFID (radio frequency identification) ou NFC (Near Field Communication).

Il est envisageable que la chaussure 1 intègre également une puce de radiocommunication longue portée comme par exemple une puce de radiocommunication GSM, qui pourrait n'être utilisée qu'en cas d'absence de communication avec les autres modules de radiocommunication, et en cas de situation très critique, du fait de la forte consommation énergétique d'une telle puce GSM.

Il est à noter que les différents composants électroniques précités peuvent également se retrouver sous la forme d'un seul ou de plusieurs composants SiP (System-in-package) ou SoC (System-on-Chip).

La chaussure 1 intègre intérieurement une batterie 3 d'alimentation électrique placée dans la semelle 10, et plus spécifiquement noyée dans sa couche d'isolation, notamment au niveau du talon. La batterie 3 est raccordée à la carte électronique 2, et en particulier au centre de traitement numérique 20 qui gère la batterie 3 pour alimenter les différents composants électroniques de la chaussure 1. Il est également envisageable de prévoir un module de gestion de la batterie 3 qui est externe au centre de traitement numérique.

La batterie 3 est de préférence du type rechargeable, soit de manière filaire (une prise extérieure étant alors prévue sur la chaussure 1), soit sans contact, par exemple selon une technologie par induction comme la technologie Qi, soit au moyen d'un système de récupération d'énergie (autrement appelé « energy harvesting » en anglais) qui convertit des mouvements du corps humain (et notamment des mouvements de marche ou de course) en énergie électrique.

En alternative, la batterie 3 n'est pas rechargeable et doit donc être remplacée lorsque vide via une trappe prévue dans la semelle 10, et notamment dans le talon.

Dans l'exemple de la figure 1, la batterie 3 est rechargeable sans contact avec une technologie par induction, de sorte que la batterie 3 est reliée à au moins une antenne ou bobine 30 placée dans la semelle 10. Ainsi, la recharge de la batterie 3 s'effectue en plaçant la chaussure 1 à proximité ou sur une station émettrice de recharge par induction (non illustrée), cette station émettrice de recharge incluant au moins une bobine primaire d'induction propre à générer un courant induit de recharge dans la au moins une bobine 30 par induction mutuelle.

La chaussure 1 intègre intérieurement au moins un capteur de force 4, du type capteur ou transducteur piézoélectrique, qui est disposé sur une face interne de la partie supérieure de chaussage 11, à l'intérieur de la chaussure 1, afin de pouvoir détecter un effort appliqué par le pied sur le capteur de force 4 ; un tel effort (ou pression) appliqué par le pied sur le capteur de force 4 se traduisant par une variation de la résistivité électrique au sein du capteur de force 4.

Le ou chaque capteur de force 4 est raccordé au module de prétraitement et de filtrage de la carte électronique 2, afin de prétraiter et filtrer les données de mesure (variations de la résistivité) de ce capteur de force 4, puis ce module de prétraitement et de filtrage adresse un signal prétraité et filtré au centre de traitement numérique 20, et plus spécifiquement au microprocesseur, afin de le traiter comme décrit ultérieurement. Le ou chaque capteur de force 4 est alimenté électriquement par la batterie 3 via le centre de traitement numérique 20.

Le ou chaque capteur de force 4 est placé à l'avant de la chaussure 1, au-dessus des orteils du pied, et notamment sur le côté intérieur de la chaussure 1 afin d'être au-dessus du gros orteil du pied. De manière classique, et en particulier dans les chaussures de sécurité, les orteils du pied ne sont pas en contact compressif avec la partie supérieure de chaussage 11, de sorte que les orteils du pied peuvent être en léger contact avec le capteur de force 4 en situation normale, voire ne pas être en contact, et seul un mouvement désiré par le porteur (qui soulève son gros orteil ou tous ses orteils) assurera une détection de l'effort appliqué sur le(s) capteur(s) de force 4. Dans le cas d'une chaussure 1 du type chaussure de sécurité avec coque de sécurité 13, le ou chaque capteur de force 4 est disposé sous cette coque de sécurité 13.

Un étalonnage préalable du ou de chaque capteur de force 4 et/ou la sélection d'un seuil minimal de détection peuvent être prévus pour éviter que de trop faibles contacts du pied sur le(s) capteur(s) de force 4 (par exemple en situation de marche) ne soient perçus comme des efforts désirés du porteur pour transmettre un message (autrement appelés des faux positifs).

Pour réduire au maximum les faux positifs (et donc économiser la batterie 3 et éviter des sollicitations erronées des services externes), il est prévu le module de prétraitement et de filtrage qui constitue un dispositif électronique de mise en forme recevant en entrée les données de mesure du capteur de force 4 correspondant (en l'occurrence la variation de la résistivité électrique), et générant en sortie un signal prétraité et filtré, autrement dit mis en forme, destiné au centre de traitement numérique 20, un tel module de prétraitement et de filtrage pouvant par exemple être du type comparateur à deux seuils ou circuit à bascule à hystérésis, tel qu'un Trigger de Schmitt.

La chaussure 1 intègre intérieurement plusieurs avertisseurs du type vibreur 50 placés à l'intérieur de la chaussure 1, et en particulier placés sur la face interne de la partie supérieure de chaussage 11, afin d'émettre chacun un signal d'avertissement vibratoire (autrement appelé signal haptique) apte à être ressenti par le pied. Ces vibreurs 50 sont reliés au centre de traitement numérique 20, et notamment au microcontrôleur, qui les pilote indépendamment les uns des autres, et ils sont alimentés électriquement par la batterie 3 via le centre de traitement numérique 20.

Comme visible sur la figure 1, les vibreurs 50 sont placés respectivement à l'avant (au niveau des orteils), à l'arrière (au niveau du talon) et sur les côtés (à droite et à gauche) de la chaussure 1 afin d'émettre des signaux d'avertissement vibratoires respectifs aptes à être ressentis par différentes zones du pied (orteil(s), talon, côtés intérieur et extérieur). Ces différents vibreurs 50 permettent ainsi un maillage géospatial en deux dimensions (avant, arrière, droite et gauche).

Il est également envisageable de prévoir un vibreur placé dans la semelle 10 en-dessous du pied et un autre vibreur placé au-dessus du pied (par exemple dans la languette 12), permettant ainsi un maillage géospatial en trois dimensions (avant, arrière, droite, gauche, haut et bas).

De manière optionnelle, la chaussure 1 intègre également d'autres avertisseurs aptes à émettre des signaux d'avertissement à destination du porteur, comme par exemple :
- un avertisseur du type source lumineuse 51 placée sur l'extérieur de la chaussure 1 afin d'émettre un signal d'avertissement lumineux apte à être vu par le porteur, cette source lumineuse 51 pouvant par exemple être placée sur le dessus de la languette 12 et être du type diode électroluminescente RGB permettant différentes colorimétrie de la lumière ; et/ou
- un avertisseur du type source sonore 52 afin d'émettre un signal d'avertissement sonore apte à être entendu par le porteur, cette source sonore 52 pouvant par exemple être placée sur le dessus de la languette 12 et être du type buzzer ou bipeur (pour émettre un signal sonore simple) ou du type haut-parleur (pour émettre des signaux vocaux) ; et/ou
- un avertisseur du type source odorante (non illustrée) afin d'émettre un signal d'avertissement odorant apte à être senti olfactivement par le porteur.

Ces avertisseurs 51, 52 sont reliés au centre de traitement numérique 20 qui les pilote indépendamment les uns des autres, et ils sont alimentés électriquement par la batterie 3 via le centre de traitement numérique 20.

De manière optionnelle, la chaussure 1 intègre également un détecteur de présence (non illustré) du pied à l'intérieur de la chaussure 1. Ce détecteur de présence pourrait être un capteur à boucle magnétique, un photodétecteur ou capteur de flux lumineux, un capteur électromécanique, etc. Ce détecteur de présence est relié au centre de traitement numérique 20 qui reçoit ainsi l'information de la présence ou de l'absence du pied dans la chaussure 1.

De manière optionnelle, la chaussure 1 intègre un ou plusieurs boutons 53 reliés au centre de traitement numérique 2, et qui peuvent être déclenchés manuellement par le porteur pour différents usages (appairage, envoi manuel d'une alerte, remise à zéro du centre de traitement numérique, etc.).

Il pourrait également être envisagé d'intégrer à la chaussure 1 des capteurs, comme par exemple un ou plusieurs capteurs choisis dans la liste suivante : accéléromètre, capteur d'orientation, gyroscope, magnétomètre, capteur d'humidité, capteur de température, capteur de pression du pied sur la semelle, capteur de gaz (notamment capteur de gaz à base d'encre comprenant des nanoparticules ayant des propriétés de détection), capteur d'image (appareil photographique ou caméra vidéo). Bien entendu, ce ou ces capteurs seront connectés au centre de traitement numérique 20 qui réceptionnera les données de mesure correspondantes.

Grâce aux vibreurs 50, au(x) capteur(s) de force 4 et à la puce de radiocommunication longue portée 23, tous connectés au centre de traitement numérique 20, la chaussure 1 permet au porteur de communiquer de manière bidirectionnelle avec des services externes (membre de son équipe, supérieur hiérarchique, responsable de la sécurité, service public de sécurité, ...), car le centre de traitement numérique 20 est, d'une part, apte à piloter la puce de radiocommunication longue portée 23 pour émettre un message (appelé signal porteur) suite à la détection d'une séquences d'efforts successifs sur le capteur de force 4 et, d'autre part, apte à piloter les avertisseurs 50, 51, 52 pour émettre un signal d'avertissement suite à la réception d'un signal externe par la puce de radiocommunication longue portée 23 ; de sorte que :
- le porteur peut émettre des messages plus ou moins complexes en exerçant des séquences d'efforts successifs sur le capteur de force 4 qui seront traduites par le centre de traitement numérique 20 en messages intelligibles (appelés signaux porteurs) qui seront transmis sans fil par la puce de radiocommunication longue portée 23 ;
- le porteur peut recevoir des messages (appelés signaux externes) plus ou moins complexes réceptionnés par la puce de radiocommunication longue portée 23, qui seront convertis par le centre de traitement numérique 20 en des motifs d'émission d'au moins un signal d'avertissement (signal vibratoire et/ou signal lumineux et/ou signal sonore et/ou signal odorant) émis par les avertisseurs 50, 51, 52 concernés.

De manière avantageuse, pour une communication des services externes vers le porteur, seuls les vibreurs 50 peuvent être activés afin de transmettre des séquences vibratoires (séquences haptiques) comprenant des vibrations successives compréhensibles par le porteur, en jouant sur l'intensité des vibrations, la durée des vibrations, l'espacement temporel des vibrations, la sélection des vibreurs 50 activés entre les vibreurs à l'avant, à l'arrière, à droite, à gauche et éventuellement en haut et en bas.

A ce double titre, le centre de traitement numérique 20 intègre :
- une première table de conversion configurée pour convertir des séquences prédéfinies d'efforts successifs appliqués par le pied sur le capteur de force 4 en des signaux porteurs respectifs associés à des messages contextuels ou intelligibles par les services externes ;
- une seconde table de conversion configurée pour convertir des signaux externes prédéfinis (en provenance des services externes) en des motifs respectifs d'émission d'au moins un signal d'avertissement.

Ces tables de conversion peuvent être paramétrables afin que le porteur puisse définir des raccourcis pour les séquences d'efforts successifs appliqués par le pied sur le capteur de force 4 et des raccourcis pour les motifs d'émission d'au moins un signal d'avertissement.

Pour réduire au maximum les faux positifs, il est prévu :
- avec le module de prétraitement et de filtrage, un pré-traitement et filtrage logiciel du signal provenant du ou de chaque capteur de force 4, sur la base d'une séquence à durée minimale et maximale (autrement dit ce module de prétraitement et de filtrage vérifie si la durée du signal est comprise dans une fourchette de temps donnée) ; et
- avec le centre de traitement numérique 20, et plus spécifiquement avec le microprocesseur, un traitement du signal provenant du module de prétraitement et de filtrage, consistant à comparer ce dernier avec des séquences enregistrées dans la première table de conversion.

Il est à noter l'intérêt d'associer, pour le centre de traitement numérique 20, au moins un microprocesseur (en particulier un microprocesseur intégrant une unité de gestion mémoire hardware ou MMU pour « Memory Management Unit ») et au moins un microcontrôleur, afin que le microprocesseur effectue les traitements numériques complexes et fortement consommateurs en énergie électrique (notamment le traitement et l'envoi des données issues du ou des capteur(s) de force 4 et le pilotage des avertisseurs) et que le microcontrôleur effectue les tâches simples et faiblement consommateurs en énergie électrique (notamment la réception de données en provenance de capteurs externes).

Il est également à noter que la chaussure 1 du pied droit (respectivement du pied gauche) d'un porteur pourrait communiquer avec la chaussure du pied gauche (respectivement du pied droit) du même porteur, afin de répartir entre les deux chaussures 1 une partie du centre de traitement numérique 20 (par exemple le microprocesseur dans la chaussure de droite et le microcontrôleur dans la chaussure de gauche, ou inversement, ou bien un type de module de radioccomunication dans la chaussure de droite et un autre type de module de radioccomunication dans la chaussure de gauche), permettant ainsi une répartition des ressources électroniques et donc du poids entre les deux chaussures.

En référence à la figure 2, la chaussure 1 s'intègre dans une installation 6 de communication bidirectionnelle comprenant au moins une telle chaussure 1, ainsi qu'un serveur central 60 distant et une passerelle 61 de liaison entre la chaussure 1 et le serveur central 60.

La passerelle 61, du type gateway ou nanoserveur, est en liaison avec :
- la puce de radiocommunication longue portée 23 de la chaussure 1 sur un réseau LPWAN ou LTN 71 ; et
- le serveur central 60 via un réseau haut débit 72 à haute ou moyenne consommation, comme exemple un réseau filaire Ethernet ou un réseau sans fil 3G, 4G, 5G ou Wi-Fi, voire Li-Fi.

L'installation 6 peut également comprendre un ou plusieurs appareils connectés 62, où le ou chaque appareil connecté 62 est équipé :
- d'au moins un capteur de mesure d'un paramètre (comme par exemple un paramètre physique, chimique, environnemental ou un paramètre d'état d'une machine) et/ou d'au moins un actionneur ; et
- un module de radiocommunication bidirectionnelle apte à émettre des données de mesure de son capteur et/ou recevoir des données de pilotage de son actionneur.

Le module de radiocommunication de l'appareil connecté 62 est en liaison avec la passerelle 61 via un réseau haut débit 73 à haute ou moyenne consommation, comme exemple un réseau filaire Ethernet ou un réseau sans fil 3G, 4G, 5G ou Wi-Fi, voire Li-Fi, de sorte que l'appareil connecté 62 est en liaison avec la chaussure 1 via la passerelle 61.

L'appareil connecté 62 peut également être en liaison directe avec la chaussure 1 via un réseau LPWAN ou LTN 74, sous réserve que l'appareil connecté 62 intègre une puce de radiocommunication longue portée adaptée, et sous réserve que la chaussure 1 se trouve à portée de l'appareil connecté 62 dans ce réseau LPWAN ou LTN 74.

Le serveur central 60 peut également être relié à un serveur de service public 63 (comme par exemple la police, l'armée, les pompiers, les services de secours, ...) via un réseau haut débit 75 à haute ou moyenne consommation, comme exemple un réseau filaire Ethernet ou un réseau sans fil 3G, 4G, 5G ou Wi-Fi, voire Li-Fi.

Le serveur central 60 peut aussi être relié à un serveur client 64 (accessible par exemple par une entreprise qui souhaite échanger avec ses employés sur le terrain) via un réseau haut débit 76 à haute ou moyenne consommation, comme exemple un réseau filaire Ethernet ou un réseau sans fil 3G, 4G, 5G ou Wi-Fi, voire Li-Fi.

La chaussure 1 peut également être reliée à un terminal mobile 65 de radiocommunication (comme par exemple un terminal téléphonique de type smartphone, une tablette numérique ou un assistant numérique personnel (PDA)) via un réseau WBAN ou WBASN 77 (ou par une connexion NFC de courte-portée qui implique la présence d'une puce NFC à l'intérieur de la chaussure 1) ; ce terminal mobile 65 étant à ce titre muni d'une puce de radiocommunication courte portée selon la technologie WBAN ou WBASN (voire également une puce NFC). En outre, ce terminal mobile 65 peut quant à lui être relié au serveur central 60 via un réseau sans fil 78 haut débit, comme exemple un réseau 3G, 4G, 5G ou Wi-Fi, voire Li-Fi.

Le terminal mobile 65 peut par exemple servir à paramétrer les tables de conversion employées par le centre de traitement numérique 20 de la chaussure 1, à recevoir des informations de la part du centre de traitement numérique 20 sur le statut des différents composants électroniques qui équipent la chaussure 1.

La chaussure 1 peut également être reliée à un dispositif portatif 66 (comme par exemple un vêtement, un casque, un bracelet, une arme, un outil, etc.) porté par le porteur de la chaussure 1, via un réseau WBAN ou WBASN 79 ; ce dispositif portatif 66 étant à ce titre muni d'une puce de radiocommunication courte portée selon la technologie WBAN ou WBASN.

Ce dispositif portatif 66 peut intégrer au moins un capteur de mesure d'un paramètre, comme par exemple :
- un paramètre physique (eg. accélération, déplacement, orientation, vitesse) ;
- un paramètre chimique (eg. détection d'un gaz) ;
- un paramètre environnemental (eg. humidité ambiante, température ambiante) ;
- un paramètre physiologique (eg. glycémie, pression artérielle, électrocardiogramme, encéphalogramme, électromyogramme, oxymétrie, transpiration).

Ainsi, le centre de traitement numérique 20 de la chaussure 1 peut recevoir des données de mesure en provenance du ou des capteurs du dispositif portatif 66, afin de les faire suivre au serveur central 60 (la chaussure servant alors de passerelle de liaison) ou de les traiter (par exemple par comparaison avec un seuil minimal et/ou un seuil maximal) pour ensuite générer un signal d'alerte à destination du serveur central 60.

En variante ou en complément, ce dispositif portatif 66 peut intégrer une étiquette de radio-identification courte-portée (par exemple étiquette passive RFID, étiquette active RFID, ou puce NFC) stockant un identifiant et éventuellement d'autres données. Ainsi, le centre de traitement numérique 20 de la chaussure 1 peut recevoir les identifiants des dispositifs portatifs 66, afin de les faire suivre au serveur central 60 pour traitement (la chaussure servant alors de passerelle de liaison) ou de les traiter lui-même, pour ensuite générer un signal d'avertissement à destination du porteur, pour lui signaler qu'il a omis ou oublié de s'équiper de tel ou tel dispositif portatif et/ou qu'un dispositif portatif est non conforme et/ou qu'un dispositif portatif est non adapté à la zone dans laquelle il se trouve à un moment donné ou non adapté à au moins une consigne prédéfinie (par exemple consigne de sécurité, consigne d'hygiène, consigne de maintenance industrielle ou consigne d'installation).

Par ailleurs, bien que non illustré sur la figure 2, la chaussure 1 peut être en liaison directe avec une autre chaussure 1 (c'est-à-dire une chaussure 1 porté par une autre porteur) via un réseau LPWAN ou LTN, sous réserve que l'autre chaussure 1 se trouve à portée de la chaussure 1 dans ce réseau LPWAN ou LTN. A ce titre, les modules de radiocommunication 23 de plusieurs chaussures 1 peuvent former un réseau maillé local, ou réseau Mesh, en mettant en oeuvre une topologie de maillage où les noeuds du réseau sont les modules de radiocommunication 23 qui sont reliés les uns avec au moins une partie des autres de manière décentralisée.

Il est également envisageable que la chaussure 1 soit en liaison avec une autre chaussure 1 via la passerelle 61.

De plus, le centre de traitement numérique 20 peut recevoir des données de détecteurs internes à la chaussure 1 qui seront analysées de sorte que, sous certaines conditions prédéfinies associées aux données de mesure du ou de chaque détecteur, le centre de traitement numérique 20 active au moins un avertisseur 50, 51, 52 pour avertir le porteur d'un danger par rapport à ces conditions prédéfinies, sans nécessiter de radiocommunication avec un tiers externe. Par exemple, le détecteur est un dispositif de géo-localisation (par exemple un système GPS) ou un détecteur de proximité (par exemple de technologie RFID) placé dans la chaussure 1 et les conditions prédéfinies correspondent à une géo-localisation ou une détection de présence dans une zone de danger.

La suite de la description concerne un procédé de communication bidirectionnelle avec une chaussure 1, au sein d'une installation 6, un tel procédé comprenant différentes phases de communication entre les différents composants de l'installation 6.

Dans toutes les phases de communication énumérées ci-dessous, la communication se fait entre le premier porteur dont le pied P est dans une première chaussure 1, le centre de traitement numérique 20 de la première chaussure 1, et un ou plusieurs tiers externes ou acteurs de l'installation 6.

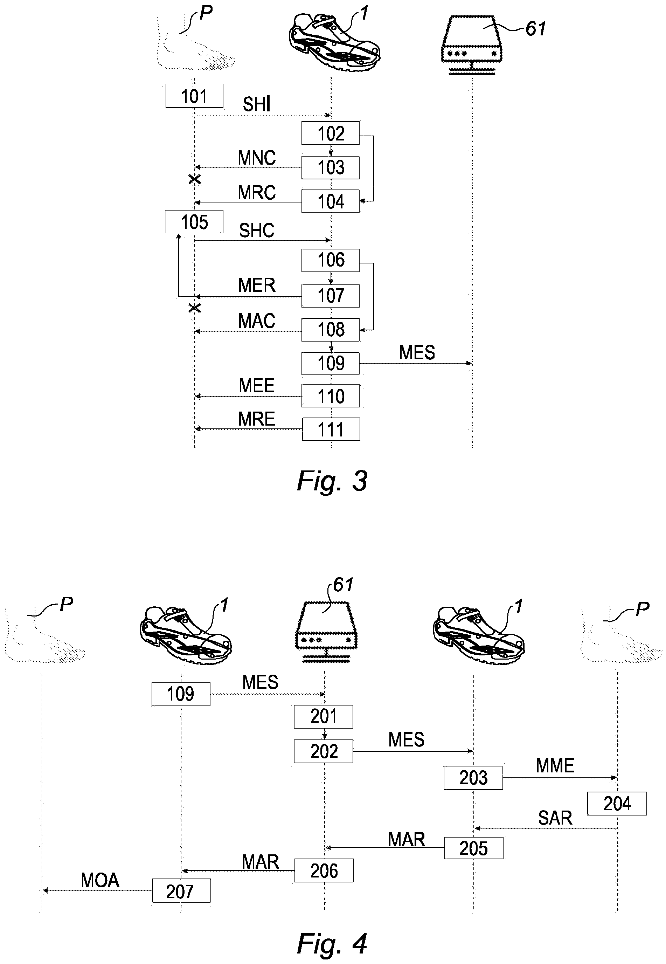

Par exemple, en référence à la figure 3, une première phase de communication pourrait être établie entre un premier porteur dont le pied P est dans une première chaussure 1, le centre de traitement numérique 20 de la première chaussure 1 et une ou plusieurs passerelles 61.

Par soucis de lisibilité, chaque phase de communication énumérée ci-dessous concerne la communication entre le premier porteur dont le pied P est dans une première chaussure 1, le centre de traitement numérique 20 de la première chaussure, et un unique acteur de l'installation 6, mais il est bien entendu que les phases de communication peuvent être établies en parallèle pour autoriser la communication du premier porteur avec plusieurs acteurs de l'installation 6.

En référence à la figure 3, une première phase de communication entre un premier porteur dont le pied P est dans une première chaussure 1, la première chaussure 1 et une première passerelle 61 est décrite ci-après.

Dans une première étape 101, le premier porteur effectue avec son pied P une séquence d'efforts successifs sur le(s) capteur(s) de force 4 afin de communiquer un message (comme par exemple un message d'alerte), cette séquence d'efforts successifs étant appelée séquence haptique initiale SHI et étant reçue par la chaussure 1 (et plus précisément reçu par le(s) capteur(s) de force). La séquence haptique initiale SHI peut être du type séquence haptique de message d'alerte SHA (afin de signaler une alerte) ou du type séquence haptique de message SHM (afin d'adresser un message, cette séquence pouvant être une séquence de raccourci de message ou une séquence de message en morse et/ou dans un autre alphabet ou code de langage qui pourrait être configuré dans le centre de traitement numérique 20).

Dans une deuxième étape 102, le centre de traitement numérique 20 de la première chaussure 1 reçoit un signal de détection SDI de la séquence haptique initiale SHI (après prétraitement et filtrage des données de mesure du ou des capteurs de force 4), et ce centre de traitement numérique 20 analyse ce signal de détection SDI et recherche si ce dernier correspond à une séquence prédéfinie (ou préconfigurée) présente dans la première table de conversion ; les séquences enregistrées dans la première table de conversion formant pour rappel des raccourcis pour des messages intelligibles.

Si le centre de traitement numérique 20 ne reconnaît pas le signal de détection SDI comme correspondant à une séquence prédéfinie, alors, dans une troisième étape 103, le centre de traitement numérique 20 pilote au moins un avertisseur 50, 51, 52 (et notamment uniquement un ou plusieurs vibreurs 50 pour des raisons de discrétion), afin de générer un motif spécifique d'émission d'au moins un signal d'avertissement, appelé motif de non compréhension MNC, qui sera compris par le premier porteur comme signifiant que la première chaussure 1 n'a pas compris sa séquence haptique initiale SHI.

Si le centre de traitement numérique 20 reconnaît le signal de détection SDI comme correspondant à une séquence SEQ prédéfinie, alors, dans une quatrième étape 104, le centre de traitement numérique 20 pilote au moins un avertisseur 50, 51, 52, afin de générer un motif spécifique d'émission d'au moins un signal d'avertissement, appelé motif de reconnaissance MRC, qui sera compris par le premier porteur comme signifiant que la première chaussure 1 a compris sa séquence haptique initiale SHI.

Ensuite, dans une cinquième étape 105, le premier porteur ayant compris que sa séquence haptique initiale SHI a été comprise ou reconnue par le centre de traitement numérique 20, il doit confirmer sa demande en refaisant la même séquence haptique initial SHI, si cette séquence haptique initial SHI est du type séquence haptique de message d'alerte SHA, afin de limiter les faux positifs. Ainsi, dans la cinquième étape 105, le premier porteur effectue avec son pied P une séquence haptique de confirmation SHC qui doit être identique à la séquence haptique initial SHI, et ce dans le cas où il souhaite émettre un message d'alerte.

Dans le cas où le premier porteur souhaitait transmettre un message informatif n'ayant pas de caractère alarmant, il aurait réalisé dans la première étape une séquence haptique initiale SHI du type séquence haptique de message SHM pour signaler au centre de traitement numérique 20 qu'il compte émettre un message informatif. Dans la cinquième étape, le premier porteur ayant compris que sa séquence haptique initiale SHI a été comprise ou reconnue par le centre de traitement numérique 20, il n'a pas besoin de confirmer sa demande, et peut alors effectuer avec son pied P une séquence haptique libre de message informatif sous la forme d'un raccourci de message préenregistré ou d'une séquence en alphabet morse et/ou dans un autre alphabet ou code de langage.

Il peut être prévu un temps maximum pour cette cinquième étape, autrement dit le premier porteur à un temps maximum pour confirmer sa demande (ou pour réaliser sa séquence haptique libre de message informatif), sinon le centre de traitement numérique 20 annule la prise en considération de la séquence haptique initiale SHI. Il est également envisageable que, si ce temps maximum est dépassé, le centre de traitement numérique 20 pilote au moins un avertisseur 50, 51, 52, afin de générer un motif spécifique d'émission d'au moins un signal d'avertissement, appelé motif d'erreur MER, qui sera compris par le premier porteur comme signifiant qu'aucune séquence haptique n'a été détectée dans le temps imparti et que la phase de communication s'arrête ici.

Dans une sixième étape 106, le centre de traitement numérique 20 reçoit un signal de détection SDC de la séquence haptique de confirmation SHC (après prétraitement et filtrage des données de mesure du ou des capteurs de force 4), dans le cas où la séquence haptique initial SHI est du type séquence haptique de message d'alerte SHA, et le compare avec la séquence SEQ prédéfinie associée à la séquence haptique initiale SHI.

Si la séquence haptique initial SHI était du type séquence haptique de message SHM alors, dans cette sixième étape 106, le centre de traitement numérique 20 reçoit un signal de détection SDC de la séquence libre de message informatif (après prétraitement et filtrage des données de mesure du ou des capteurs de force 4), et le décode avec sa première table de conversion qui intègre les raccourcis de message, ainsi que l'alphabet morse et/ou un autre alphabet ou code de langage.

Si le centre de traitement numérique 20 ne reconnaît pas le signal de détection SDC comme correspondant à la séquence SEQ ou comme correspondant à une séquence de message informatif (selon un raccourci préenregistré ou selon un alphabet préenregistré), alors, dans une septième étape 107, le centre de traitement numérique 20 pilote au moins un avertisseur 50, 51, 52, afin de générer un motif spécifique d'émission d'au moins un signal d'avertissement, appelé motif d'erreur MER, qui sera compris par le premier porteur comme signifiant que la séquence haptique de confirmation SHC est différente de la séquence haptique initiale SHI ou n'a pas été comprise, et que donc sa demande ne sera pas traitée. De manière avantageuse, le premier porteur dispose d'au moins une autre possibilité pour refaire une séquence haptique de confirmation SHC qui soit identique à la séquence haptique initiale SHI, ou pour refaire une séquence haptique libre de message informatif, encore avec un temps maximum pour s'exécuter.

Si ce temps maximum est dépassé, le centre de traitement numérique 20 pilote au moins un avertisseur 50, 51, 52, afin de générer un motif spécifique d'émission d'au moins un signal d'avertissement, appelé motif d'erreur MER, qui sera compris par le premier porteur comme signifiant qu'aucune séquence de détection SDC n'a été reçue dans le temps imparti et que la phase de communication s'arrête ici.

Si le centre de traitement numérique 20 reconnaît que le signal de détection SDC correspond à la séquence SEQ ou à une séquence de message informatif, alors, dans une huitième étape 108, le centre de traitement numérique 20 pilote au moins un avertisseur 50, 51, 52, afin de générer un motif spécifique d'émission d'au moins un signal d'avertissement, appelé motif d'accord MAC, qui sera compris par le premier porteur comme signifiant que la séquence haptique de confirmation SHC est identique à la séquence haptique initiale SHI (dans le cas où la séquence haptique initial SHI était du type séquence haptique de message d'alerte SHA) ou a bien été comprise (dans le cas où la séquence haptique initial SHI était du type séquence haptique de message SHM), et que donc sa demande va pouvoir être traitée et envoyée par la première chaussure 1 à destination d'un ou plusieurs tiers externes, acteurs de l'installation 6 illustrée sur la figure 2.

Dans une neuvième étape, le centre de traitement numérique 20 envoie à la première passerelle 61 (qui est à portée sur le réseau LPWAN ou LTN) un message MES associé à la séquence SEQ dans la première table de conversion, autrement dit associé à la séquence haptique initiale SHI, ou bien associé à la séquence haptique libre de message informatif.

En cas d'échec de l'envoi du message MES, alors, dans une dixième étape 110, le centre de traitement numérique 20 pilote au moins un avertisseur 50, 51, 52, afin de générer un motif spécifique d'émission d'au moins un signal d'avertissement, appelé motif d'échec envoi MEE, qui sera compris par le premier porteur comme signifiant que la première chaussure 1 échoue à envoyer le message. Par défaut, l'envoi du message MES est prioritaire pour le centre de traitement numérique 20, de sorte que le centre de traitement numérique 20 essaie indéfiniment d'envoyer le message MES et, selon la configuration choisie, le motif d'échec envoi MEE est mis en oeuvre à intervalles réguliers pour informer le premier porteur du non envoi du message MES.

En cas de réussite de l'envoi du message MES, alors, dans une onzième étape 111, le centre de traitement numérique 20 pilote au moins un avertisseur 50, 51, 52, afin de générer un motif spécifique d'émission d'au moins un signal d'avertissement, appelé motif de réussite envoi MRE, qui sera compris par le premier porteur comme signifiant que la chaussure 1 a réussi à envoyer le message.

En référence à la figure 4, une deuxième phase de communication, qui fait suite à la première phase de communication décrite ci-dessus en référence à la figure 3, consiste en une communication entre le premier porteur dont le pied P est dans la première chaussure 1, la première chaussure 1, la première passerelle 61, un second porteur dont le pied P est dans une seconde chaussure 1 et la seconde chaussure 1, et est décrite ci-après. Dans cette deuxième phase, la seconde chaussure 1 est à portée de la première passerelle 61 sur le réseau LPWAN ou LTN.

Après réception du message MES en provenance de la première chaussure 1, dans une première étape 201, la première passerelle 61 vérifie grâce à une base de données locale si des autres chaussures 1 (donc des autres porteurs) sont à portée pour recevoir le message MES, permettant ainsi un temps de traitement de la demande plus rapide, car sans passer par le serveur central 60.

Dans une deuxième étape 202, la première passerelle 61 ayant détecté une seconde chaussure 1 à portée, elle redirige le message MES à destination de la seconde chaussure 1 à portée.

Dans une troisième étape 203, après réception du message MES, le centre de traitement numérique 20 de la seconde chaussure 1 pilote au moins un avertisseur 50, 51, 52, afin de générer un motif spécifique d'émission d'au moins un signal d'avertissement, appelé motif de message MME, qui sera compris par le second porteur en l'associant au message MES adressé par le premier porteur. Autrement dit, le second porteur reçoit dans sa seconde chaussure 1 ce motif de message MME, et sait le traduire en une demande ou une information intelligible.

Pour attirer l'attention du second porteur, le motif de message MME peut être précédé par un motif de réveil (par exemple une longue vibration suivie d'une pause) afin que le second porteur puisse se concentrer sur le motif de message MME qui suit.

Dans une quatrième étape 204, le second porteur, ayant compris la demande du premier porteur, confirme la bonne réception et la bonne compréhension du motif de message MME en effectuant avec son pied P une séquence d'efforts successifs sur le(s) capteur(s) de force 4 qui correspond à une séquence haptique d'accusé réception SAR pour signaler la bonne prise en compte de la demande du premier porteur. Tant que le second porteur n'a pas effectué la séquence haptique d'accusé réception SAR, le motif de message MME est répété à intervalles réguliers sur sa seconde chaussure 1. Il est à noter que cette séquence haptique d'accusé réception SAR peut également contenir une sous-séquence SSI spécifique relative à des instructions et/ou informations destinées au premier porteur.

Dans une cinquième étape 205, le centre de traitement numérique 20 de la seconde chaussure 1 reçoit un signal de détection SDAR de la séquence haptique d'accusé réception SAR (après prétraitement et filtrage des données de mesure du ou des capteurs de force 4), et le convertit en un message d'accusé réception MAR, avant de le transmettre à la première passerelle 61. En cas de sous-séquence SSI, le message d'accusé réception MAR contiendra des instructions et/ou informations destinées au premier porteur.

Dans une sixième étape 206, la première passerelle 61 redirige le message d'accusé réception MAR à destination de la première chaussure 1 à portée.

Dans une septième étape 207, le centre de traitement numérique 20 de la première chaussure 1 reçoit le message d'accusé réception MAR et ensuite pilote au moins un avertisseur 50, 51, 52, afin de générer un motif spécifique d'émission d'au moins un signal d'avertissement, appelé motif d'accusé réception MOA, qui sera compris par le premier porteur comme signifiant qu'au moins un destinataire de sa demande a bien reçu et compris sa demande, et a acquiescé la réception de celle-ci, éventuellement accompagné d'instructions et/ou informations.

Un traitement des différents message d'accusé de réception MAR est fait afin d'éviter que le premier porteur ne reçoive plusieurs messages d'accusé de réception MAR pour le même message MES, ce traitement s'appliquant à fois sur les messages d'accusé de réception MAR avec ou sans instructions et/ou informations.

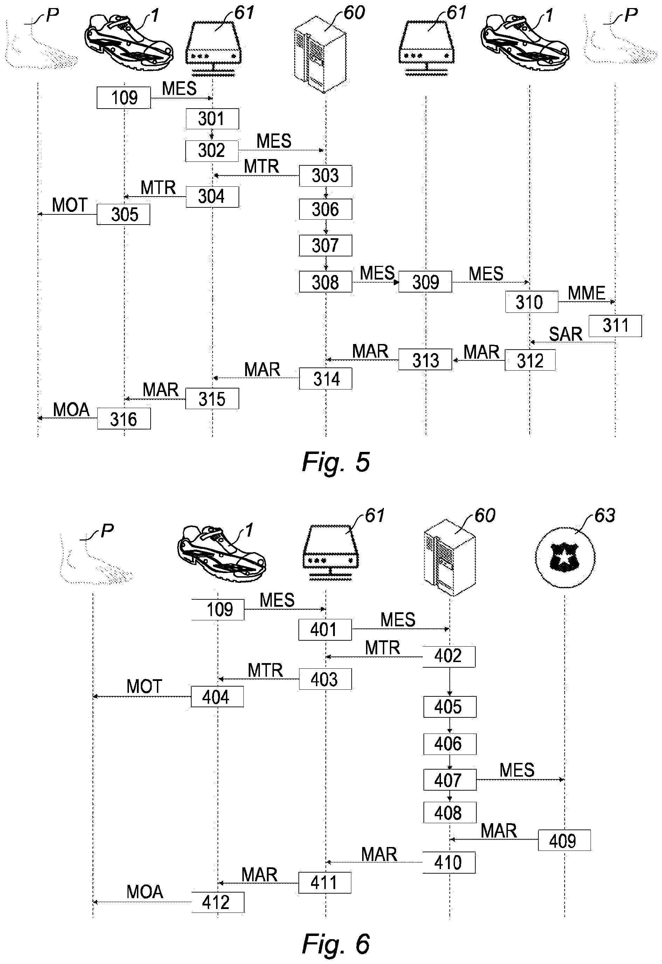

En référence à la figure 5, une troisième phase de communication, qui fait suite à la première phase de communication décrite ci-dessus en référence à la figure 3, consiste en une communication entre le premier porteur dont le pied P est dans la première chaussure 1, la première chaussure 1, la première passerelle 61, le serveur central 60, une seconde passerelle 61, un second porteur dont le pied P est dans une seconde chaussure 1 et la seconde chaussure 1, et est décrite ci-après. Dans cette troisième phase, la seconde chaussure 1 n'est pas à portée de la première passerelle 61 sur le réseau LPWAN ou LTN, et est donc accessible uniquement via le serveur central 60 et via une seconde passerelle 61 qui est quant à elle à portée de la seconde chaussure 1.

Après réception du message MES en provenance de la première chaussure 1, dans une première étape 301, la première passerelle 61 vérifie grâce à une base de données locale si des autres chaussures 1 (donc des autres porteurs) sont à portée pour recevoir le message MES. Dans le cas présent, toutes les chaussures 1 devant recevoir le message MES ne sont pas à portée.

Dans une deuxième étape 302, la première passerelle 61 n'ayant pas détecté que toutes les chaussures 1 devant recevoir le message MES sont à portée, elle redirige le message MES à destination du serveur central 60. Il est bien entendu que cette troisième phase peut s'effectuer en parallèle de la seconde phase, afin que les chaussures 1 à portée de la première passerelle 61 comme les chaussures 1 qui ne sont pas à portée de la première passerelle 61 puissent recevoir la demande du premier porteur.

Dans une troisième étape 303, le serveur central 60 réceptionne le message MES et transmet à la première passerelle 61 un message de traitement MTR signifiant qu'il traite la demande du premier porteur.

Dans une quatrième étape 304, la première passerelle 61 redirige le message de traitement MTR à destination de la première chaussure 1 à portée.

Dans une cinquième étape 305, le centre de traitement numérique 20 de la première chaussure 1 reçoit le message de traitement MTR et ensuite pilote au moins un avertisseur 50, 51, 52, afin de générer un motif spécifique d'émission d'au moins un signal d'avertissement, appelé motif de traitement MOT, qui sera compris par le premier porteur comme signifiant que sa demande a bien été reçu et est en cours de traitement.

Dans une sixième étape 306, le serveur central 60 vérifie si des options de gestion ont été fixées par un gestionnaire de l'installation 6 sur la redirection du message MES à tel ou tel service externe (un service public, un responsable de la sécurité, un responsable hiérarchique, ...), et notamment vérifie s'il est nécessaire d'obtenir un accord de la part d'un responsable de rang 1 (par exemple le gestionnaire ou un supérieur hiérarchique) avant de rediriger le message MES. Dans ce cas, il est mis en place un échange entre le responsable de rang 1 et le serveur central 60 afin de recueillir son accord.

Parmi ces options de gestion figurent également la possibilité de requérir l'accord d'un responsable de rang 2 si le responsable de rang 1 n'a pas répondu au bout d'un délai donné, et ainsi de suite avec un responsable de rang 3, etc.

Dans une septième étape 307, si l'accord d'un responsable est reçu ou si un tel accord est non nécessaire dans les options de gestion, alors le serveur central 60 recherche quel destinataire final (eg. un autre porteur, un service public, un responsable ...) de l'installation 6 doit être prévenu de la demande du premier porteur.

Lors de cette recherche, il peut être prévu un filtre pour sélectionner le ou les destinataires finaux (eg. un autre porteur, un service public, ...) du message MES, ce filtre pouvant être automatisé (selon le type ou contenu du message MES) et/ou pouvant être manuel en étant exprimé par le responsable qui donne son accord.

Dans cette troisième phase, le destinataire final est un second porteur, étant entendu qu'il pourrait aussi s'agir d'un service public (voir la quatrième phase de communication décrite ci-après en référence à la figure 6).

Dans une huitième étape 308, le serveur central 60 ayant détecté un second porteur accessible via une seconde passerelle 61, le serveur central 60 redirige le message MES à destination de la seconde passerelle 61.

Ensuite, les étapes 309 à 312 correspondent substantiellement aux étapes 202 à 205 décrites précédemment où, à la fin de la douzième étape 312, le centre de traitement numérique 20 de la seconde chaussure 1 transmet le message d'accusé réception MAR à la seconde passerelle 61.

Lors des étapes 313 à 315, le message d'accusé réception MAR est redirigé à destination de la première chaussure 1 via la seconde passerelle 61, le serveur central 60 et la première passerelle 61, et la seizième étape 316 correspond substantiellement à l'étape 207 avec la génération du motif d'accusé réception MOA.

En référence à la figure 6, une quatrième phase de communication, qui fait suite à la première phase de communication décrite ci-dessus en référence à la figure 3, consiste en une communication entre le premier porteur dont le pied P est dans la première chaussure 1, la première chaussure 1, la première passerelle 61, le serveur central 60 et un serveur de service public 63.

Après réception du message MES en provenance de la première chaussure 1, dans une première étape 401, la première passerelle 61 redirige le message MES à destination du serveur central 60. Bien entendu, l'étape 301 aurait pu être présente, étant rappelé que cette quatrième phase peut se dérouler en parallèle de la troisième phase et/ou de la deuxième phase.

Les étapes 402 à 405 correspondent substantiellement aux étapes 303 à 306 décrites précédemment.

La sixième étape 406 correspond substantiellement à l'étape 307, à la différence que le destinataire final sélectionné est un serveur de service public 63, dans le cadre de cette quatrième phase de communication.

Dans une septième étape 407, le serveur central 60 redirige le message MES à destination du serveur de service public 63 et, dans une huitième étape 408, le serveur central 60 sauvegarde l'envoi de ce message MES au serveur de service public 63. Le message MES pourra être préalablement formaté pour s'insérer au mieux dans le système d'informations du service public 63.

Dans une neuvième étape 409, le serveur de service public 63 réceptionne le message MES et retourne au serveur central 60 un message d'accusé réception MAR, qui peut éventuellement contenir des instructions et/ou informations destinées au premier porteur.

Lors des étapes 410 et 411, le message d'accusé réception MAR est redirigé à destination de la première chaussure 1 via le serveur central 60 et la première passerelle 61, et la douzième étape 412 correspond substantiellement à l'étape 207 avec la génération du motif d'accusé réception MOA.

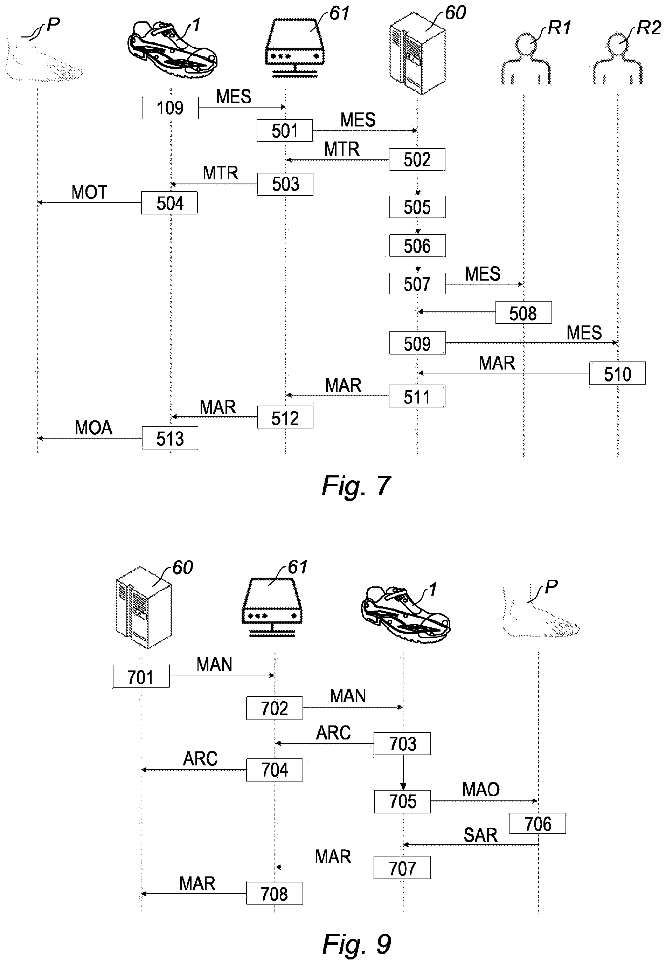

En référence à la figure 7, une cinquième phase de communication, qui fait suite à la première phase de communication décrite ci-dessus en référence à la figure 3, consiste en une communication entre le premier porteur dont le pied P est dans la première chaussure 1, la première chaussure 1, la première passerelle 61, le serveur central 60 et un ou plusieurs responsables R1, R2, tels que des responsables hiérarchiques, gestionnaires, responsables de la sécurité.

Après réception du message MES en provenance de la première chaussure 1, dans une première étape 501, la première passerelle 61 redirige le message MES à destination du serveur central 60. Bien entendu, l'étape 301 aurait pu être présente, étant précisé que cette cinquième phase peut se dérouler en parallèle des deuxième, troisième et/ou quatrième phases.

Les étapes 502 à 505 correspondent substantiellement aux étapes 303 à 306 décrites précédemment.

La sixième étape 506 correspond substantiellement à l'étape 307, à la différence que le destinataire final sélectionné est un responsable R1, dans le cadre de cette cinquième phase de communication. Il peut s'agir d'un responsable de la sécurité ou d'un responsable hiérarchique présent (ou non) sur le site où se trouve le premier porteur.

Dans une septième étape 507, le serveur central 60 ayant détecté un responsable R1, le serveur central 60 redirige le message MES à destination de ce responsable R1, qui peut la recevoir par le biais d'une chaussure 1 (et on revient aux seconde et troisième phases de communication décrites précédemment), d'une application mobile, d'un message textuel ou vocal sur terminal mobile ou informatique.

Dans une huitième étape 508, le responsable R1 réceptionne le message MES et retourne au serveur central 60 un message d'accusé réception MAR, qui peut éventuellement contenir des instructions et/ou informations destinées au premier porteur.

En l'absence de réception du message d'accusé réception MAR de la part du responsable R1 (par exemple après plusieurs envois du message MES et/ou après un délai donné), dans une neuvième étape 509, le serveur central 60 recherche un autre responsable R2 et redirige le message MES à destination de cet autre responsable R2, qui lui aussi doit retourner un message d'accusé réception MAR dans une dixième étape 510.

Lors des étapes 511 et 512, le message d'accusé réception MAR est redirigé à destination de la première chaussure 1 via le serveur central 60 et la première passerelle 61, et la treizième étape 513 correspond substantiellement à l'étape 207 avec la génération du motif d'accusé réception MOA.

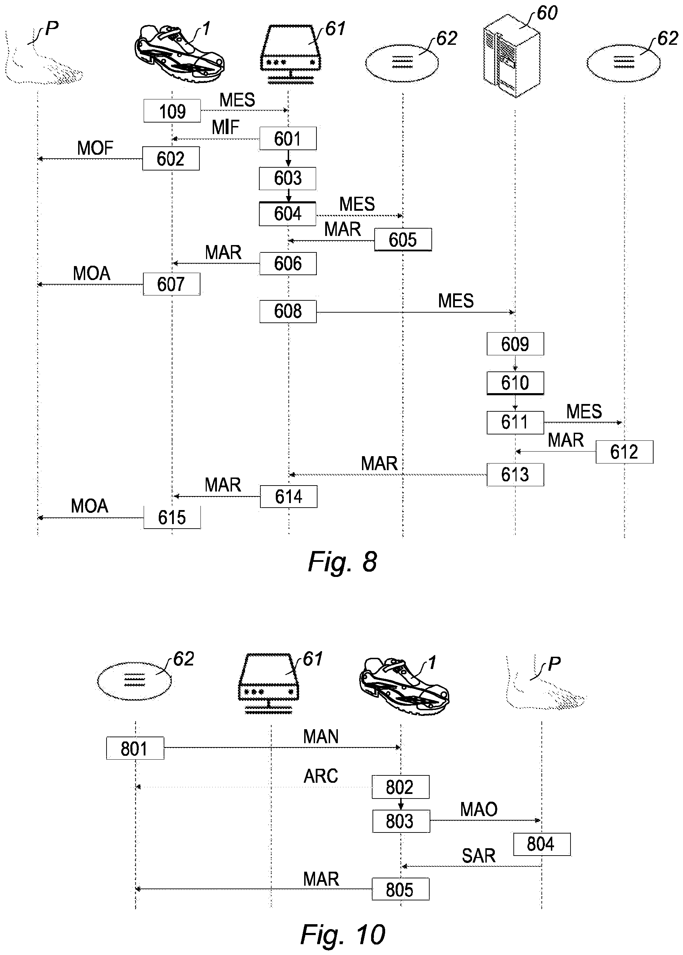

En référence à la figure 8, une sixième phase de communication, qui fait suite à la première phase de communication décrite ci-dessus en référence à la figure 3, consiste en une communication entre le premier porteur dont le pied P est dans la première chaussure 1, la première chaussure 1, la première passerelle 61, un premier appareil connecté 62 local accessible via la première passerelle 61 ou un second appareil connecté 62 distant accessible via le serveur central 60.

Après réception du message MES en provenance de la première chaussure 1, dans une première étape 601, la première passerelle 61 analyse le message MES qu'elle interprète comme devant être transmis en priorité à un appareil connecté 62 (par exemple une sirène, une aspiration de gaz, etc.), et elle adresse un message d'information MIF afin d'informer le premier porteur que son message est en cours de traitement.

Dans une deuxième étape 602, le centre de traitement numérique 20 de la première chaussure 1 reçoit le message d'information MIF et ensuite pilote au moins un avertisseur 50, 51, 52, afin de générer un motif spécifique d'émission d'au moins un signal d'avertissement, appelé motif d'information MOF, qui sera compris par le premier porteur comme signifiant que son message est en cours de traitement.

Dans une troisième étape 603, la première passerelle 61 vérifie, dans une base de données locale, si des appareils connectés 62 destinés à recevoir le message MES sont à portée sur un réseau LPWAN ou LTN, pour un traitement rapide du message.

Dans une quatrième étape 604, ayant détecté un premier appareil connecté 62 sur son réseau LPWAN ou LTN, la première passerelle 61 redirige le message MES à destination du premier appareil connecté 62.

Dans une cinquième étape 605, le premier appareil connecté 62 réceptionne le message MES et retourne à la première passerelle 61 un message d'accusé réception MAR, et les étapes 606 et 607 correspondent substantiellement aux étapes 206 et 207.

Dans une huitième étape 608, n'ayant pas détecté d'appareil connecté 62 sur son réseau LPWAN ou LTN, la première passerelle 61 redirige le message MES à destination du serveur central 60.

Dans une neuvième étape 609, le serveur central 60 vérifie si des options de gestion ont été fixées par un gestionnaire de l'installation 6 sur la redirection du message MES à tel ou tel appareil connecté 62, de la même manière que dans l'étape 306, et vérifie notamment s'il est nécessaire d'obtenir un accord d'un responsable avant de rediriger le message MES.

Dans une dixième étape 610, si l'accord d'un responsable est reçu ou si un tel accord est non nécessaire dans les options de gestion, alors le serveur central 60 recherche quel appareil connecté 62 de l'installation 6 doit être prévenu de la demande du premier porteur, en fonction par exemple de la teneur du message MES, ou bien d'un lien entre le premier porteur et l'appareil connecté 62 (lien d'équipe, lien d'entreprise, ...).

Dans une onzième étape 611, le serveur central 60 ayant détecté un second appareil connecté 62 à prévenir, le serveur central 60 redirige le message MES à destination de ce second appareil connecté 62 (soit directement soit via une seconde passerelle 61).

Dans une douzième étape 612, le second appareil connecté 62 réceptionne le message MES et retourne au serveur central 60 un message d'accusé réception MAR.

Lors des étapes 613 et 614, le message d'accusé réception MAR est redirigé à destination de la première chaussure 1 via le serveur central 60 et la première passerelle 61, et la quinzième étape 615 correspond substantiellement à l'étape 207 avec la génération du motif d'accusé réception MOA.

Dans les septième et huitième phases de communication suivantes, la communication concerne un serveur central 60 ou un appareil connecté 62 ou un service public 63 et un ou plusieurs acteurs de l'installation 6. Par exemple, en référence à la figure 9, la phase de communication se fait entre un serveur central 60 et un ou plusieurs porteurs de chaussure 1 via une ou plusieurs passerelles 61. Par soucis de lisibilité, les septième et huitième phases de communication énumérées ci-dessous concerneront la communication entre un premier porteur d'une première chaussure 1 et un serveur central 60 (septième phase de communication de la figure 9) ou un appareil connecté 62 (huitième phase de communication de la figure 10) ; mais ces phases de communication restent valables dans le cas d'autres acteurs de l'installation 6 ou de plusieurs acteurs de l'installation 6.

En référence à la figure 9, une septième phase de communication consiste en une communication entre le serveur central 60 et le porteur de la première chaussure 1 via la première passerelle 61.

Dans une première étape 701, le serveur central 60 transmet un message d'annonce MAN à destination de la première passerelle 61, ce message d'annonce MAN étant destiné au porteur de la première chaussure 1 qui est en liaison sur réseau LPWAN ou LTN avec la première passerelle 61.

Ce message d'annonce MAN peut avoir été édité par un gestionnaire ou autre responsable, par exemple au moyen d'une interface type terminal informatique ou terminal mobile, afin d'adresser des informations et/ou des instructions, étant entendu que ce message d'annonce MAN peut être destiné à d'autres porteurs de chaussures 1.

Dans une deuxième étape 702, la première passerelle 61 ayant détecté la première chaussure 1 à portée, elle redirige le message d'annonce MAN à destination de la première chaussure 1.

Dans une troisième étape 703, le centre de traitement numérique 20 de la première chaussure 1 réceptionne le message d'annonce MAN et retourne à la première passerelle 61 un message d'accusé réception chaussure ARC qui indique que la première chaussure 1 a bien réceptionné le message d'annonce MAN.

Dans une quatrième étape 704, la première passerelle 61 redirige le message d'accusé réception chaussure ARC à destination du serveur central 60.

Dans une cinquième étape 705, après réception du message d'annonce MAN, le centre de traitement numérique 20 de la première chaussure 1 pilote au moins un avertisseur 50, 51, 52, afin de générer un motif spécifique d'émission d'au moins un signal d'avertissement, appelé motif d'annonce MAO, qui sera compris par le premier porteur en l'associant au message d'annonce MAN adressé par le serveur central 60. Autrement dit, le premier porteur reçoit dans sa première chaussure 1 ce motif d'annonce MAO, et sait le traduire en une information et/ou instruction intelligible.

Pour attirer l'attention du premier porteur, le motif d'annonce MAO peut être précédé par un motif de réveil (par exemple une longue vibration suivie d'une pause) afin que le premier porteur puisse se concentrer sur le motif d'annonce MAO qui suit.

Dans une sixième étape 706, le premier porteur, ayant compris l'information et/ou instruction, confirme la bonne réception et la bonne compréhension du motif d'annonce MAO en effectuant avec son pied P une séquence d'efforts successifs sur le(s) capteur(s) de force 4 qui correspond à une séquence haptique d'accusé réception SAR. Tant que le premier porteur n'a pas effectué la séquence haptique d'accusé réception SAR, le motif de message d'annonce MAO est répété à intervalles réguliers sur sa première chaussure 1. Il est à noter que cette séquence haptique d'accusé réception SAR peut également contenir une sous-séquence SSI spécifique relative à des instructions et/ou informations destinées au serveur central 60.

Dans une septième étape 707, le centre de traitement numérique 20 de la première chaussure 1 reçoit un signal de détection SDAR de la séquence haptique d'accusé réception SAR (après prétraitement et filtrage des données de mesure du ou des capteurs de force 4), et le convertit en un message d'accusé réception MAR, avant de le transmettre à la première passerelle 61. Dans une huitième étape 708, la première passerelle 61 redirige le message d'accusé réception MAR à destination du serveur central 60.

En référence à la figure 10, une huitième phase de communication consiste en une communication entre un appareil connecté 62 (voire également un service public 63 bien que non illustré) et le porteur de la première chaussure 1.

Dans une première étape 801, l'appareil connecté 62 (ou un service public 63) transmet un message d'annonce MAN à destination de la première chaussure 1 qui se situe à sa portée dans un réseau LPWAN ou LTN, ce message d'annonce MAN étant destiné au(x) porteur(s) afin par exemple de transmettre une alerte propre à l'appareil connecté 62 (ou service public 63) comme : « détection d'un gaz dangereux » si l'appareil connecté est un détecteur de gaz, ou « dysfonctionnement interne, éloignez vous », ou « vous être trop proche d'une zone de danger ».

Les étapes 802 à 805 correspondent substantiellement aux étapes 703 à 707 décrite ci-dessus, à la différence que, dans la troisième étape 703 et dans la cinquième étape 805, le message d'accusé réception chaussure ARC et le message d'accusé réception MAR sont transmis à l'appareil connecté 62 (ou au service public 63).

Dans une variante non illustrée, les différents messages MAN, ARC et MAR peuvent transiter par une première passerelle 61 si la première chaussure 61 n'est pas accessible par l'appareil connecté sur un réseau LPWAN ou LTN.

De manière générale, et en particulier pour la mise en oeuvre des septième et huitième phases décrites ci-dessus, en référence aux figures 9 et 10, il est bien évident que le centre de traitement numérique 20 de la chaussure 1 est dans une phase d'écoute afin de réceptionner tout message entrant, et notamment le message d'annonce MAN, sans qu'il soit nécessaire que la chaussure 1 ait émis précédemment un message vers l'extérieur.

Selon une première réalisation, le centre de traitement numérique 20 de la chaussure 1 est dans une phase d'écoute continue, sans interruption ni pause, c'est-à-dire que le centre de traitement numérique 20 ouvre en continu au moins un canal de communication entrant pour la réception de tout message entrant qui lui est adressé par l'extérieur. Dans cette première réalisation, l'écoute est dite synchrone, dans la mesure où la réception du message entrant par le centre de traitement numérique 20 est synchronisée sur l'émission du message entrant par l'émissaire externe (personne ou machine), aux temps de radiocommunication près.

Selon une seconde réalisation, le centre de traitement numérique 20 de la chaussure 1 alterne successivement entre :
- des phases d'écoute durant lesquelles le centre de traitement numérique 20 ouvre au moins un canal de communication entrant pour la réception de tout message entrant qui lui est adressé par l'extérieur ;
- des phases de pause durant lesquels le centre de traitement numérique 20 ferme son ou tous ses canaux de communication entrant, principalement à des fins d'économie énergétique.

Les durées des phases d'écoute et des phases de pause sont paramétrables, par exemple les phases de pause peuvent durer entre 1 et 10 minutes, voire plus, selon le contexte du porteur de la chaussure 1 et selon les capacités de la batterie 3.

Dans cette seconde réalisation, le centre de traitement numérique 20 réceptionne les messages entrants selon un protocole désynchronisé ou asynchrone comme suit :
- durant les phases de pause, tout message entrant adressé de l'extérieur est dirigé et stocké dans une mémoire de messagerie d'un serveur distant, comme par exemple le serveur central 60 ou un autre serveur dédié à cette tâche ; et
- durant les phases d'écoute, le centre de traitement numérique 20 établit une liaison avec cette mémoire de messagerie et relève le ou les messages entrant stockés.

Dans cette seconde réalisation, l'écoute est dite asynchrone, dans la mesure où la réception du message entrant par le centre de traitement numérique 20 est désynchronisée par rapport à l'émission du message entrant par l'émissaire externe (personne ou machine).

Ainsi, au travers des différentes phases de communication, il est évident qu'une communication bidirectionnelle, intelligible et complexe peut être établie entre le porteur d'une chaussure 1 et différents services externes, tels qu'un serveur central 60, des personnes physiques, un serveur de service public, un appareil connecté, ...

En exploitant le(s) capteur(s) de force 4 et le(s) vibreur(s) de la chaussure 1, il est possible, en toute discrétion, de communiquer de manière bidirectionnelle et haptique avec le porteur de la chaussure 1.

Il est à noter que l'invention peut trouver des applications dans d'autres domaines que celui de la sécurité ou de la surveillance d'alerte, comme par exemple comme :
- solution de communication pour une personne en situation d'handicap en exploitant un accessoire vestimentaire intégrant au moins un capteur de force sur lequel une partie du corps de cette personne peut agir (suivant le handicap), ou
- solution d'interface haptique pour jeux vidéo, en utilisant par exemple un gant comme accessoire vestimentaire ;
- solution d'interaction sensorielle pour stimuler des parties sensibles du corps humains entre deux adultes distants, en utilisant par exemple un sous-vêtement comme accessoire vestimentaire.

## Revendications

1. Article vestimentaire (1) intégrant au moins un module de radiocommunication (23, 24) bidirectionnelle apte à émettre et recevoir des signaux, au moins un capteur de force (4) placé sur l'article vestimentaire (1) et apte à détecter un effort appliqué par un porteur de l'article vestimentaire (1) sur le au moins un capteur de force (4), au moins un avertisseur (50, 51, 52) apte à émettre un signal d'avertissement à destination au moins dudit porteur, au moins une batterie (3) électrique, et un centre de traitement numérique (20) reprogrammable et configurable qui est raccordé audit au moins un module de radiocommunication (23), audit au moins un capteur de force (4), audit au moins un avertisseur (50, 51, 52) et à ladite batterie (3), ledit centre de traitement numérique (20) étant apte à piloter au moins un avertisseur (50, 51, 52) pour émettre un signal d'avertissement (MNC, MRC, MER, MAC, MEE, MRE, MME, MOA, MOT, MAO, MOF) suite à la réception d'un signal externe (MES, MTR, MAR, MAN, MIF) par ledit au moins un module de radiocommunication (23 ; 24) ou suite à la détection d'au moins une séquence d'efforts successifs (SHI, SHC) sur ledit au moins un capteur de force (4) ; dans lequel le centre de traitement numérique (20) est apte à piloter ledit au moins
un module de radiocommunication (23) pour émettre un signal porteur (MES, MAR), suite à la détection d'au moins une séquence d'efforts successifs (SHI, SHC, SAR) sur ledit capteur de force (4),
et ledit au moins un avertisseur (50, 51, 52) comprend au moins un avertisseur du type vibreur (50) placé à l'intérieur de l'article vestimentaire (1) afin d'émettre un signal d'avertissement vibratoire apte à être ressenti par le porteur ;
ledit article vestimentaire (1) étant **caractérisé en ce que** le centre de traitement numérique (2) intègre une première table de conversion configurée pour convertir des séquences prédéfinies d'efforts successifs (SHI, SHC, SAR) appliqués par le porteur sur le capteur de force (4) en des signaux porteurs (MES, MAR) respectifs associés à des messages ;
et **en ce que** le centre de traitement numérique (2) intègre une seconde table de conversion configurée pour convertir des signaux externes (MES, MTR, MAR, MAN, MIF) prédéfinis en des motifs (MME, MOT, MOA, MAO, MOF) respectifs d'émission d'au moins un signal d'avertissement vibratoire.

2. Article vestimentaire (1) selon l'une quelconque des revendications précédentes, dans lequel la première table de conversion est du type paramétrable.

3. Article vestimentaire (1) selon l'une quelconque des revendications précédentes, dans lequel la seconde table de conversion est du type paramétrable.

4. Article vestimentaire (1) selon l'une quelconque des revendications précédentes, dans lequel l'article vestimentaire (1) est une chaussure (1) comprenant une semelle (10) et une partie supérieure de chaussage (11) pourvue d'une entrée pour l'introduction d'un pied (P) d'un porteur.

5. Article vestimentaire (1) selon la revendication 4, dans lequel le ou chaque capteur de force (4) est positionné sur une face interne de la partie supérieure de chaussage (11) et à l'avant de la chaussure (1), de sorte que ledit ou chaque capteur de force (4) est placé au-dessus d'au moins un orteil du pied (P).

6. Article vestimentaire (1) selon la revendication 5, comprenant en outre une coque de sécurité (13) placée à l'avant de la chaussure (1), où le ou chaque capteur de force (4) est disposé sous ladite coque de sécurité.

7. Article vestimentaire (1) selon l'une quelconque des revendications précédentes, dans lequel le centre de traitement numérique (20) alterne successivement entre :
- des phases d'écoute durant lesquelles le centre de traitement numérique (20) ouvre au moins un canal de communication entrant pour la réception de tout message entrant qui lui est adressé par l'extérieur ;
- des phases de pause durant lesquels le centre de traitement numérique (20) ferme son ou tous ses canaux de communication entrant.

8. Installation (6) de communication bidirectionnelle avec au moins un porteur d'un article vestimentaire (1), ladite installation (6) comprenant au moins un article vestimentaire (1) conforme à l'une quelconque des revendications précédentes, un serveur central (60) distant et au moins une passerelle (61) de liaison entre un module de radiocommunication (23) de l'article vestimentaire (1) et le serveur central (60).

9. Installation (6) selon la revendication 8, dans laquelle le module de radiocommunication (23) de l'article vestimentaire (1) est en liaison avec un module de radiocommunication (23) d'au moins un autre article vestimentaire (1) conforme à l'une quelconque des revendications 1 à 7, soit directement soit par l'intermédiaire d'une passerelle (61).

10. Installation (6) selon la revendication 9, dans laquelle les modules de radiocommunication (23) de plusieurs articles vestimentaires (1) forment un réseau maillé local dont les noeuds sont lesdits modules de radiocommunication (23) qui sont reliés directement les uns avec au moins une partie des autres de manière décentralisée.

11. Installation (6) selon l'une quelconque des revendications 8 à 10, comprenant en outre au moins un appareil connecté (62) équipé d'au moins un capteur de mesure d'un paramètre, notamment du type paramètre physique, chimique ou environnemental, et/ou d'au moins un actionneur, ledit appareil connecté (62) comprenant en outre un module de radiocommunication bidirectionnelle apte à émettre des données de mesure de son capteur et/ou recevoir des données de pilotage de son actionneur, et dans laquelle le module de radiocommunication (23) de l'article vestimentaire (1) est en liaison avec le module de radiocommunication du au moins un appareil connecté (62), soit directement soit par l'intermédiaire de la passerelle (61).

12. Procédé de communication bidirectionnelle avec un article vestimentaire (1) conforme à l'une quelconque des revendications 1 à 7, comprenant les étapes suivantes :
- détection d'une séquence d'efforts successifs (SHI, SHC, SAR) effectuée par un porteur de l'article vestimentaire (1) sur le au moins un capteur de force (4) ;
- réception par le centre de traitement numérique (20) d'un signal de détection de ladite séquence d'efforts successifs (SHI, SHC, SAR) par le au moins un capteur de force (4), après un éventuel prétraitement et un filtrage des signaux issus directement du au moins un capteur de force (4) ;
- analyse par le centre de traitement numérique (20) dudit signal de détection afin de vérifier s'il est associé à un signal porteur (MES, MAR) prédéfini correspondant à un message intelligible ;
- pilotage par le centre de traitement numérique (20) d'au moins un avertisseur (50, 51, 52) pour émettre un signal d'avertissement (MNC, MRC, MER, MAC, MEE, MRE) suite à la réception dudit signal de détection, ledit pilotage comprenant le pilotage d'au moins un avertisseur du type vibreur (50) placé à l'intérieur de l'article vestimentaire (1) pour émettre un signal d'avertissement vibratoire apte à être ressenti par le porteur ;
- pilotage par le centre de traitement numérique (20) du module de radiocommunication (23) pour émettre ledit signal porteur (MES, MAR) ;
- réception d'un signal externe (MES, MTR, MAR, MAN, MIF) par le module de radiocommunication (23), et transmission dudit signal externe au centre de traitement numérique (20) ;
- pilotage par le centre de traitement numérique (20) d'au moins un avertisseur (50, 51, 52) pour émettre un signal d'avertissement (MME, MOA, MOT, MAO, MOF) suite à la réception dudit signal externe (MES, MTR, MAR, MAN, MIF), ledit pilotage comprenant le pilotage d'au moins un avertisseur du type vibreur (50) placé à l'intérieur de l'article vestimentaire (1) pour émettre un signal d'avertissement vibratoire apte à être ressenti par le porteur.

13. Procédé de communication bidirectionnelle selon la revendication 12, dans lequel le centre de traitement numérique (20) alterne successivement entre :
- des phases d'écoute durant lesquelles le centre de traitement numérique (20) ouvre au moins un canal de communication entrant pour la réception de tout message entrant qui lui est adressé par l'extérieur ;
- des phases de pause durant lesquels le centre de traitement numérique (20) ferme son ou tous ses canaux de communication entrant

14. Procédé de communication bidirectionnelle selon les revendications 12 ou 13, comprenant les étapes successives suivantes :
- durant la ou une phase d'écoute, le centre de traitement numérique (20) réceptionne le message d'annonce (MAN) en provenance d'un émissaire externe ;
- le centre de traitement numérique (20) pilote ledit au moins un avertisseur (50 51, 52), afin de générer un motif d'annonce (MAO) spécifique d'émission dudit au moins un signal d'avertissement ;
- le centre de traitement numérique (20) réceptionne un signal de détection (SDAR) issu dudit au moins un capteur de force (4) suite à la réalisation, par le porteur de l'article vestimentaire (1), d'une séquence d'efforts successifs sur le au moins un capteur de force (4) et qui correspond à une séquence haptique d'accusé réception (SAR) ;
- le centre de traitement numérique (20) convertit le signal de détection (SDAR) en un message d'accusé réception (MAR) ;
- le centre de traitement numérique (20) communique à l'émissaire externe le message d'accusé réception (MAR).

## Patentansprüche

1. Kleidungsstück (1), einschließlich mindestens eines bidirektionalen Funkkommunikationsmoduls (23, 24), das zum Senden und Empfangen von Signalen ausgelegt ist, mindestens eines Kraftsensors (4), der an dem Kleidungsstück (1) angeordnet ist und zum Detektieren einer Kraft, die durch einen Träger des Kleidungsstücks (1) auf den mindestens einen Kraftsensor (4) ausgeübt wird, ausgelegt ist, mindestens einer Warneinrichtung (50, 51, 52), die zum Senden eines Warnsignals mindestens an den Träger ausgelegt ist, mindestens einer elektrischen Batterie (3) und eines umprogrammierbaren und konfigurierbaren digitalen Verarbeitungszentrums (20), das mit dem mindestens einen Funkkommunikationsmodul (23), mit dem mindestens einen Kraftsensor (4), mit der mindestens einen Warneinrichtung (50, 51, 52) und mit der Batterie (3) verbunden ist, wobei das digitale Verarbeitungszentrum (20) dazu ausgelegt ist, nach dem Empfangen eines externen Signals (MES, MTR, MAR, MAN, MIF) durch das mindestens eine Funkkommunikationsmodul (23; 24) oder nach dem Detektieren mindestens einer Abfolge von aufeinanderfolgenden Kräften (SHI, SHC) auf den mindestens einen Kraftsensor (4) mindestens eine Warneinrichtung (50, 51, 52) zum Aussenden eines Warnsignals (MNC, MRC, MER, MAC, MEE, MRE, MME, MOA, MOT, MAO, MOF) zu steuern; wobei das digitale Verarbeitungszentrum (20) dazu ausgelegt ist, das mindestens eine Funkkommunikationsmodul (23) zu steuern, um nach der Detektion mindestens einer Abfolge von aufeinanderfolgenden Kräften (SHI, SHC, SAR) auf den Kraftsensor (4) ein Trägersignal (MES, MAR) auszusenden,
und wobei die mindestens eine Warneinrichtung (50, 51, 52) eine vibrierende Warneinrichtung (50) umfasst, die innerhalb des Kleidungsstücks (1) angeordnet ist, um ein Vibrationswarnsignal auszusenden, das so ausgelegt ist, dass der Träger es fühlen kann;
wobei das Kleidungsstück (1) **dadurch gekennzeichnet ist, dass** das digitale Verarbeitungszentrum (2) eine erste Umrechnungstabelle enthält, die so konfiguriert ist, dass sie vordefinierte Abfolgen von aufeinanderfolgenden Kräften (SHI, SHC, SAR), die vom Träger auf den Kraftsensor (4) ausgeübt werden, in entsprechende Trägersignale (MES, MAR) umwandelt, die jeweils Nachrichten zugeordnet sind;
und dadurch, dass das digitale Verarbeitungszentrum (2) eine zweite Umrechnungstabelle enthält, die so konfiguriert ist, dass sie vordefinierte externe Signale (MES, MTR, MAR, MAN, MIF) in die jeweiligen Muster (MME, MOT, MOA, MAO, MOF) für das Senden von mindestens einem Vibrationswarnsignal umwandelt.

2. Kleidungsstück (1) nach einem der vorhergehenden Ansprüche, bei dem die erste Umrechnungstabelle vom konfigurierbaren Typ ist.

3. Kleidungsstück (1) nach einem der vorhergehenden Ansprüche, bei dem die zweite Umrechnungstabelle vom konfigurierbaren Typ ist.

4. Kleidungsstück (1) nach einem der vorhergehenden Ansprüche, bei dem das Bekleidungsstück (1) ein Schuh (1) ist, der eine Sohle (10) und ein Schuhoberteil (11), der mit einem Einstieg zum Einführen eines Fußes (P) eines Trägers versehen ist, umfasst.

5. Kleidungsstück (1) nach Anspruch 4, bei dem der oder jeder Kraftsensor (4) an einer Innenseite des Schuhoberteils (11) und an der Vorderseite des Schuhs (1) angeordnet ist, so dass der oder jeder Kraftsensor (4) über mindestens einem Zeh des Fußes (P) angeordnet ist.

6. Kleidungsstück (1) nach Anspruch 5, ferner umfassend eine Sicherheitskappe (13), die an der Vorderseite des Schuhs (1) angeordnet ist, wobei der oder jeder Kraftsensor (4) unter der Sicherheitskappe angeordnet ist.

7. Kleidungsstück (1) nach einem der vorhergehenden Ansprüche, bei dem das digitale Verarbeitungszentrum (20) nacheinander wechselt zwischen:
- Abhörphasen, in denen das digitale Verarbeitungszentrum (20) mindestens einen eingehenden Kommunikationskanal für das Empfangen jeder eingehenden Nachricht öffnet, die von außen an ihn adressiert sind;
- Pausephasen, in denen das digitale Verarbeitungszentrum (20) seine oder alle seine eingehenden Kommunikationskanäle schließt.

8. Bidirektionale Kommunikationseinrichtung (6) mit mindestens einem Träger eines Kleidungsstücks (1), wobei die Einrichtung (6) mindestens ein Kleidungsstück (1) umfasst, das einem der vorhergehenden Ansprüche entspricht, mit einem entfernten zentralen Server (60) und mindestens einem Verbindungsgateway (61) zwischen einem Funkkommunikationsmodul (23) des Kleidungsstücks (1) und dem zentralen Server (60).

9. Einrichtung (6) nach Anspruch 8, bei der das Funkkommunikationsmodul (23) des Bekleidungsstücks (1) entweder direkt oder über ein Gateway (61) in Verbindung mit einem Funkkommunikationsmodul (23) mindestens eines weiteren Kleidungsstücks (1) nach einem der Ansprüche 1 bis 7 steht.

10. Einrichtung (6) nach Anspruch 9, bei der die Funkkommunikationsmodule (23) mehrerer Bekleidungsstücke (1) ein lokales Maschennetz bilden, dessen Knoten die Funkkommunikationsmodule (23) sind, die dezentral mit mindestens einem Teil der anderen direkt miteinander verbunden sind.

11. Einrichtung (6) nach einem der Ansprüche 8 bis 10, ferner umfassend mindestens ein verbundenes Gerät (62), das mit mindestens einem Sensor zum Messen eines Parameters, insbesondere der physikalischen, chemischen oder umweltbedingten Art, und/oder mindestens einer Betätigungseinrichtung ausgestattet ist, wobei das verbundene Gerät (62) ferner ein bidirektionales Funkkommunikationsmodul umfasst, das zum Senden der Messdaten von seinem Sensor und/oder zum Empfangen der Steuerdaten von seinem Aktuator ausgelegt ist, und bei dem das Funkkommunikationsmodul (23) des Kleidungsstücks (1) entweder direkt oder über das Gateway (61) mit dem Funkkommunikationsmodul mindestens eines verbundenen Geräts (62) in Verbindung steht.

12. Bidirektionales Kommunikationsverfahren mit einem Kleidungsstück (1) nach einem der Ansprüche 1 bis 7, umfassend die folgenden Schritte:
- Detektieren einer Abfolge von aufeinanderfolgenden Kräften (SHI, SHC, SAR), die durch einen Träger des Kleidungsstücks (1) auf den mindestens einen Kraftsensor (4) ausgeübt werden;
- Empfangen eines Detektionssignals der Abfolge von aufeinanderfolgenden Kräften (SHI, SHC, SAR) von dem mindestens einen Kraftsensor (4) durch das digitale Verarbeitungszentrum (20) nach einer möglichen Vorverarbeitung und eines Filterns von Signalen, die direkt von dem mindestens einen Kraftsensor (4) ausgegeben werden;
- Analysieren des Detektionssignals durch das digitale Verarbeitungszentrum (20), um zu überprüfen, ob es einem vordefinierten Trägersignal (MES, MAR) zugeordnet ist, das einer verständlichen Nachricht entspricht;
- Steuern mindestens einer Warneinrichtung (50, 51, 52) durch das digitale Verarbeitungszentrum (20), um ein Warnsignal (MNC, MRC, MER, MAC, MEE, MRE) nach dem Empfangen der Signaldetektion auszusenden, wobei das Steuern das Steuern mindestens einer vibrierenden Warneinrichtung (50) umfasst, die innerhalb des Kleidungsstücks (1) angeordnet ist, um ein Vibrationswarnsignal auszusenden, das so ausgelegt ist, dass der Träger es fühlen kann;
- Steuern des Funkkommunikationsmoduls (23) durch das digitale Verarbeitungszentrum (20), um das Trägersignal (MES, MAR) zu senden;
- Empfangen eines externen Signals (MES, MTR, MAR, MAN, MIF) durch das Funkkommunikationsmodul (23) und Übertragen des externen Signals an das digitale Verarbeitungszentrum (20);
- Steuern mindestens eines Warnsignals (50, 51, 52) durch das digitale Verarbeitungszentrum (20), um nach dem Empfangen des externen Signals (MES, MTR, MAR, MAN, MIF) ein Warnsignal (MME, MOA, MOT, MAO, MOF) zu senden, wobei das Steuern das Steuern mindestens einer vibrierenden Warneinrichtung (50) umfasst, der innerhalb des Kleidungsstücks (1) angeordnet ist, um ein Vibrationswarnsignal auszusenden, das so ausgelegt ist, dass der Träger es fühlen kann;

13. Bidirektionales Kommunikationsverfahren nach Anspruch 12, bei dem das digitale Verarbeitungszentrum (20) nacheinander wechselt zwischen:
- Abhörphasen, in denen das digitale Verarbeitungszentrum (20) mindestens einen eingehenden Kommunikationskanal für das Empfangen jeder eingehenden Nachricht öffnet, die von außen an ihn adressiert sind;
- Pausephasen, in denen das digitale Verarbeitungszentrum (20) seine oder alle seine eingehenden Kommunikationskanäle schließt.

14. Bidirektionales Kommunikationsverfahren nach Anspruch 12 oder 13, umfassend die folgenden aufeinanderfolgenden Schritte:
- während der oder einer Abhörphase empfängt das digitale Verarbeitungszentrum (20) die Ankündigungsnachricht (MAN) von einem externen Absender;
- das digitale Verarbeitungszentrum (20) steuert die mindestens eine Warneinrichtung (50, 51, 52), um ein bestimmtes Sendeankündigungsmuster (MAO) des mindestens einen Warnsignals zu erzeugen;
- das digitale Verarbeitungszentrum (20) empfängt ein Detektionssignal (SDAR), das von mindestens einem Kraftsensor (4) nach der Durchführung einer Abfolge von aufeinanderfolgenden Kräften auf den mindestens einen Kraftsensor (4) durch den Träger des Kleidungsstücks (1) ausgegeben wird und einer haptischen Abfolge einer Empfangsbestätigung (SAR) entspricht;
- das digitale Verarbeitungszentrum (20) wandelt das Detektionssignal (SDAR) in eine Empfangsbestätigungsnachricht (MAR) um;
- das digitale Verarbeitungszentrum (20) übermittelt die Empfangsbestätigungsnachricht (MAR) an den externen Sender.

## Claims

1. A garment item (1) integrating at least one bidirectional radio-communication module (23, 24) capable of emitting and receiving signals, at least one force sensor (4) placed on the garment item (1) and capable of detecting an effort applied by a wearer of the garment item (1) on the at least one force sensor (4), at least one warning device (50, 51, 52) capable of emitting a warning signal at least to said wearer, at least one electric battery (3), and a reprogrammable and configurable digital processing center (20) which is connected to said at least one radio-communication module (23), to said at least one force sensor (4), to said at least one warning device (50, 51, 52) and to said battery (3), said digital processing center (20) being capable of piloting at least one warning device (50, 51, 52) so as to emit a warning signal (MNC, MRC, MER, MAC, MEE, MRE, MME, MOA, MOT, MAO, MOF) subsequently to the reception of an external signal (MES, MTR, MAR, MAN, MIF) by said at least one radio-communication module (23, 34) or subsequently to the detection of at least one sequence of successive efforts (SHI, SHC) on said at least one force sensor (4);
wherein the digital processing center (20) is capable of piloting said at least one radio-communication module (23) so as to emit a meaningful signal (MES, MAR), subsequently to the detection of at least one sequence of successive efforts (SHI, SHC, SAR) on said force sensor (4),
and said at least one warning device (50, 51, 52) comprises at least one vibrator type warning device (50) placed inside the garment item (1) in order to emit a vibrational warning signal which can be felt by the wearer;
said garment item (1) being **characterized in that** the digital processing center (2) integrates a first conversion table configured to convert predefined sequences of successive efforts (SHI, SHC, SAR) applied by the wearer on the force sensor (4) into respective meaningful signals (MES, MAR) associated to messages;
and **in that** the digital processing center (2) integrates a second conversion table configured to convert predefined external signals (MES, MTR, MAR, MAN, MIF) into respective emission patterns (MME, MOT, MOA, MAO, MOF) of at least one vibrational warning signal.

2. The garment item (1) according to any one of the preceding claims, wherein the first conversion table is of the parameterizable type.

3. The garment item (1) according to any one of the preceding claims, wherein the second conversion table is of the parameterizable type.

4. The garment item (1) according to any one of the preceding claims, wherein the garment item (1) is a shoe (1) comprising a sole (10) and an upper stocking portion (11) provided with an entrance for the introduction of a foot (P) of a wearer.

5. The garment item (1) according to claim 4, wherein the or each force sensor (4) is positioned on an inner face of the upper stocking portion (11) and at the front of the shoe (1), so that said or each force sensor (4) is placed above at least one toe of the foot (P).

6. The garment item (1) according to claim 5, further comprising a safety shell (13) placed at the front of the shoe (1), where the or each force sensor (4) is disposed beneath said safety shell.

7. The garment item (1) according to any one of the preceding claims, wherein the digital processing center (20) switches successively between:
- listening phases during which the digital processing center (20) opens at least one entering communication channel for the reception of any entering message that is addressed thereto from the outside;
- break phases during which the digital processing center (20) closes its or all its entering communication channel(s).

8. An installation (6) for bidirectional communication with at least one wearer of a garment item (1), said installation (6) comprising at least one garment item (1) in accordance with any one of the preceding claims, a remote central server (60) and at least one gateway (61) for connection between a radio-communication module (23) of the garment item (1) and the central server (60).

9. The installation (6) according to claim 8, wherein the radio-communication module (23) of the garment item (1) is in connection with a radio-communication module (23) of at least one other garment item (1) in accordance with any one of claims 1 to 7, either directly or via a gateway (61).

10. The installation (6) according to claim 9, wherein the radio-communication modules (23) of several garment items (1) form a mesh local area network whose nodes are said radio-communication modules (23) which are connected with at least part of each other in a decentralized manner.

11. The installation (6) according to any one of claims 8 to 10, further comprising at least one connected apparatus (62) equipped with at least one sensor for measuring a parameter, such as in particular a physical, chemical or environmental parameter, and/or at least one actuator, said connected apparatus (62) further comprising a bidirectional radio-communication module capable of emitting measurement data of its sensor and/or receiving piloting data of its actuator, and wherein the radio-communication module (23) of the garment item (1) is in connection with the radio-communication module of the at least one connected apparatus (62), either directly or via the gateway (61).

12. A method for bidirectional communication with a garment item (1) in accordance with any one of claims 1 to 7, comprising the following steps:
- detection of a sequence of successive efforts (SHI, SHC, SAR) performed by a wearer of the garment item (1) on the at least one force sensor (4);
- reception by the digital processing center (20) of a detection signal of said sequence of successive efforts (SHI, SHC, SAR) by the at least one force sensor (4), after a possible pre-processing and filtering of the signals originating directly from the at least one force sensor (4);
- analysis by the digital processing center (20) of said detection signal in order to check up whether it is associated to a predefined meaningful signal (MES, MAR) corresponding to an intelligible message;
- piloting by the digital processing center (20) of at least one warning device (50, 51, 52) so as to emit a warning signal (MNC, MRC, MER, MAC, MEE, MRE) subsequently to the reception of said detection signal, said piloting comprising the piloting of at least one vibrator type warning device (50) placed inside the garment item (1) in order to emit a vibrational warning signal which can be felt by the wearer;
- piloting by the digital processing center (20) of the radio-communication module (23) so as to emit said meaningful signal (MES, MAR);
- reception of an external signal (MES, MTR, MAR, MAN, MIF) by the radio-communication module (23), and transmission of said external signal to the digital processing center (20);
- piloting by the digital processing center (20) of at least one warning device (50, 51, 52) so as to emit a warning signal (MME, MOA, MOT, MAO, MOF) subsequently to the reception of said external signal (MES, MTR, MAR, MAN, MIF), said piloting comprising the piloting of at least one vibrator type warning device (50) placed inside the garment item (1) in order to emit a vibrational warning signal which can be felt by the wearer.

13. The bidirectional communication method according to claim 12, wherein the digital processing center (20) switches successively between:
- listening phases during which the digital processing center (20) opens at least one entering communication channel for the reception of any entering message that is addressed thereto from the outside;
- break phases during which the digital processing center (20) closes its or all its entering communication channel(s).

14. The bidirectional communication method according to claims 12 or 13, comprising the following successive steps:
- during the or a listening phase, the digital processing center (20) receives the announcement message (MAN) coming from an external emitter;
- the digital processing center (20) pilots the at least one warning device (50, 51, 52), in order to generate a specific emission announcement pattern (MAO) of the at least one warning signal;
- the digital processing center (20) receives a detection signal (SDAR) originating from said at least one force sensor (4) subsequently to the completion, by the wearer of the garment item (1), of a sequence of successive efforts on the at least one force sensor (4) and which corresponds to a receipt-acknowledgement haptic sequence (SAR);
- the digital processing center (20) converts the detection signal (SDAR) into a receipt-acknowledgement message (MAR);
- the digital processing center (20) communicates the receipt-acknowledgement message (MAR) to the external emitter.
